# EUROPEAN PATENT APPLICATION

(11) **EP 1 760 091 A1**
(43) Date of publication of application: **07.03.2007**
(21) Application number: 06119636.6
(22) Date of filing: 28.08.2006
(51) Int. Cl.: C07K 14/72, C12N 15/63

(54) **Knock-out animal for TAAR1 function**

(30) Priority: 06.09.2005 EP 05108145
(71) Applicant: F. HOFFMANN-LA ROCHE AG, 4070 Basel (CH)
(72) Inventor: Hoener, Marius, 4058, Basel (CH); Lindemann, Lothar, 4056, Basel (CH); Meyer, Claas Aiko, 4056, Basel (CH); Pauly-Evers, Meike, 79111, Freiburg (DE)

(57) **Abstract**

The present invention relates to a vector construct comprising genomic sequences homologous to upstream and downstream regions flanking the single coding exon of the TAAR1 gene, one or more selection marker genes and optionally a reporter gene and the use thereof. The present invention further provides TAAR1 knock-out animals and the use thereof.

## Description

Trace amines (TAs) are endogenous compounds related to biogenic amine neurotransmitters and present in the mammalian nervous system in trace amounts. The intense research efforts on the pharmacology and metabolism of trace amines during the last decades has been triggered by their tight link to a variety of highly prevalent conditions such as depression, schizophrenia, anxiety disorders, bipolar disorder, attention deficit hyperactivity disorder, neurological diseases such as Parkinson's Disease, epilepsy, migraine, hypertension, substance abuse and metabolic disorders such as eating disorders, diabetes, obesity and dyslipidemia (Reviewed in: Lindemann & Höner, Trends Pharmacol Sci. 2005; 26(5):274-81 Branchek, T.A. and Blackburn, T.P. (2003) Curr. Opin. Pharmacol. 3, 90-97; Premont, R.T., Gainetdinov, R.R., Caron, M.G. (2001) Proc. Natl. Acad. Sci. 98, 9474-9475.; Davenport, A.P. (2003) Curr. Opin. Pharmacol. 3,127-134.). However, a detailed understanding of the physiology of trace amines on the molecular level has only become possible with the recent identification of a novel family of G protein-coupled receptors termed Trace Amine Associated Receptors (TAARs; Lindemann, L., Ebeling, M., Kratochwil, N.A., Bunzow J.R., Grandy, D.K., Hoener, M.C. (2005) Genomics 85, 372-385; Bunzow, et al. (2001) Mol. Pharmacol. 60, 1181-1188; Borowsky, B., et al., (2001). Proc. Natl. Acad. Sci. U. S. A. 98, 8966-8971). Some of these receptors display sensitivity to trace amines, and their unique pharmacology and expression pattern make these receptors prime candidates for targets in drug development in the context of several diseases, some of which previously had been linked to trace amines. Progress in understanding the physiological relevance of Trace Amine Associated Receptors and their ligands on the systems level critically depends on a detailed knowledge of their expression pattern, their pharmacology and the modes of signal transduction. In this context, compounds acting as agonists, antagonists or positive or negative modulators on TAARs, as well as transgenic animal models such as targeted "knock-out" mouse lines are essential tools for dissecting the molecular function of this receptor family and to fully understand their potential relevance as targets in drug development.

The present invention provides vector constructs and methods for producing non-human knock-out animals comprising within their genome a targeted deletion of the *TAAR1* gene. Said TAAR1 knock-out animals, as well as methods of producing them, are also provided. The invention also relates to the use of these animals as a tool for assessing KM/26.06.2006

TAAR1 function and for identifying unknown ligands of TAAR1 as well as for the characterization of novel ligands of TAARs other than TAAR1, for analyzing the tissue distribution of TAAR1, for analyzing TAAR1 signal transduction mechanisms, for analyzing the physiological function of TAAR1 *in vivo,* and for identifying and testing for the therapeutic effect of a compound in treating and preventing disorders comprising depression, anxiety disorders, bipolar disorder, attention deficit hyperactivity disorder, stress-related disorders, psychotic disorders such as schizophrenia, neurological diseases such as Parkinson's Disease, neurodegenerative disorders such as Alzheimer's disease, epilepsy, migraine, hypertension, substance abuse and metabolic disorders such as eating disorders, diabetes, diabetic complications, obesity, dyslipidemia, disorders of energy consumption and assimilation, disorders and malfunction of body temperature homeostasis, disorders of sleep and circadian rhythm, and cardiovascular disorders.

The present invention therefore provides a vector construct comprising genomic sequences homologous to upstream and downstream regions flanking the single coding exon of the TAAR1 gene suitable for homologous recombination and one or more selection marker genes.

The vector construct comprises genomic DNA sequences which are homologous to the sequences flanking the TAAR1 exon upstream and downstream on the chromosome. The lengths of the homologous sequences are chosen that it allows a targeted homologous recombination with the TAAR1 allele. The homologous sequences may have a length of 2.5 up to 300 kb. Preferably, the homologous sequences have a length of 3 to 6 kb. Preferably, the homologous sequences comprise at least a part of the TAAR1 promoter.

Preferably, the vector construct comprise additionally a reporter gene. Said reporter gene is located between the homologous TAAR1 flanking sequences. Preferably, the expression of said reporter gene is, after the integration into the genome, under the control of the TAAR1 promoter and optionally of other TAAR1 regulatory elements. The reporter gene may be selected of a group comprising LacZ or derivatives thereof, alkaline phophatase, fluorescent proteins, luciferases, or other enzymes or proteins which may be specifically detected and quantified in tissue or cells of various kinds. Preferably, the reporter gene is LacZ. Preferably, the reporter gene is at its N-terminus operably linked to a nuclear localization sequence (NLS). In a preferred embodiment, the reporter gene is inserted into the TAAR1 genomic sequence such that the endogenous start codon of the TAAR1 gene is preserved.

The selection marker gene may be a positive selection marker. The positive selection marker may be selected from the group comprising a neomycin resistance gene, a hygromycin resistance gene, a puromycin resistance gene, a blasticidin S resistance gene, a xanthine/guanine phosphoribosyl transferase gene or a zeomycin resistance gene. The positive selection marker may be framed by recognition sites for a recombinase, which allows for excision of the positive selection marker gene after selection of successful homologous recombination events. Thereby, any effect of the expression of the positive selection marker on the expression of the reporter gene may be avoided. The recognition sites for a recombinase may be selected from the group comprising frt sites for flp recombinase and loxP sites (including mutated loxP sites) for cre recombinase. Preferably, the positive selection marker is a neomycin resistance gene.

The selection marker gene may also be an negative selection marker. The negative selection marker may be selected from, but not limited to, the group consisting of a diphtheria toxin gene and an HSV-thymidine kinase gene. Preferably, the negative selection marker is a diphtheria toxin gene.

Preferably, the vector construct comprises positive selection marker and a negative selection marker. More preferably, the vector construct comprises a neomycin resistance gene and a diphtheria toxin gene.

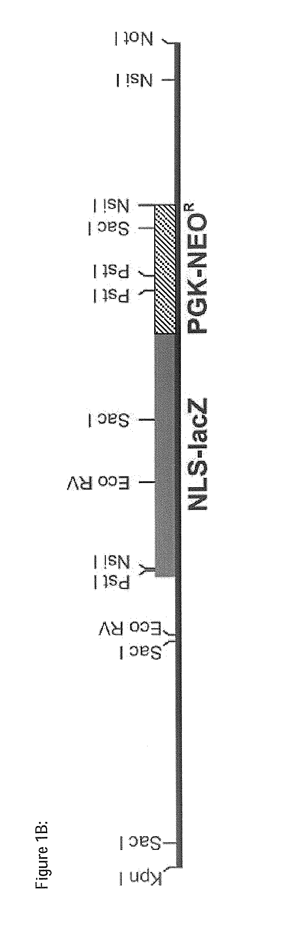

In a preferred embodiment, the vector construct is the vector contruct TAAR KO incorporated in the plasmid pSKDT-Tar1-NLS-PGK-Neo deposited under accession number DSMZ 17504 (Deposition date: 16.08.2005). Vector construct TAAR KO is depicted in Figure 1B.

The present invention further provides a method of producing a non-human knock-out animal, whose one or both alleles of *TAAR1* gene are mutated and/or truncated in a way that less or no active *TAAR1* protein is expressed comprising
(a) introducing a vector construct as described above into the genome of an embryonic stem cell by means of homologous recombination,
(b) generating a heterozygous and/or homozygous knock-out animal from the said embryonic stem cell, and thereby
(c) producing a non-human knock-out animal, whose one or both alleles of the *TAAR1* gene are mutated and/or truncated in a way that less or no active *TAAR1* protein is expressed.

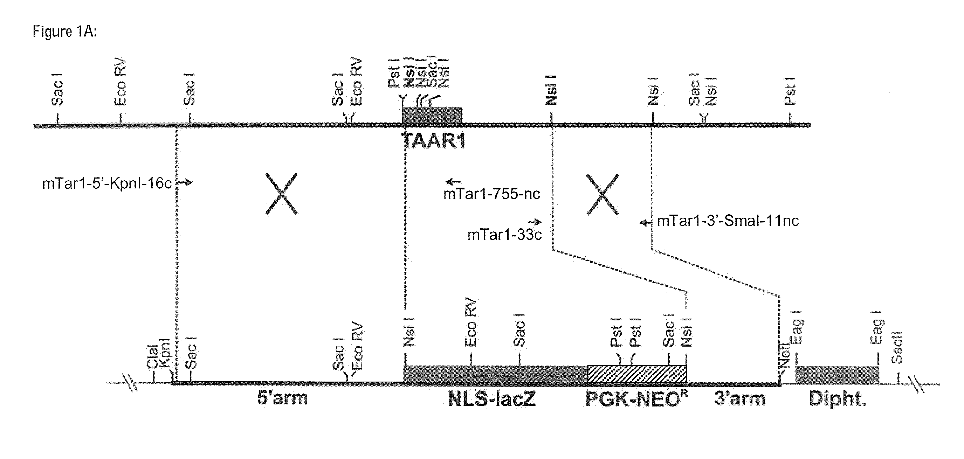

In a preferred embodiment, in step (c) of the described method, a non-human knock-out animal may be produced whose one or both alleles of a *TAAR1* gene comprise the TAAR1^{NLSlacZ} allele as depicted in Fig. 1A. In another preferred embodiment, in step (c) of the described method, a non-human knock-out animal may be produced whose one or both alleles of TAAR1 gene comprise the construct TAAR1-KO (see Fig. 1B) incorporated in the plasmid pSKDT-Tarl-NLS-PGK-Neo deposited under accession number DSMZ 17504 (Deposition date: 16.08.2005).

In a further embodiment, the above-described method additionally comprises (d) further crossbreeding the knock-out animal produced in step (c) with an animal transgenic for the recombinase recognizing the recognition sites framing the positive selection marker gene.

Knock-out animals comprising targeted mutations are achieved routinely in the art as provided for example by the method by Joyner, A.L. (Gene Targeting. 1999, Second Edition, The Practical Approach Series, Oxford University Press, New York) and Hogan, B., et al. (Manipulating the mouse embryo. 1994, Second Edition, Cold Spring Harbor Press, Cold Spring Harbor.).

For example, the heterozygous and/or homozygous knock-out animal of the above-described methods may be generated by selecting embryonic stem (ES) cell clones carrying the targeted TAAR1 allele as described above, verifying the targeted mutation in the recombinant embryonic stem cell clones, injecting the verified recombinant embryonic stem cells into blastocysts of wild type animals, transferring these injected blastocysts into pseudo-pregnant foster mothers, breeding chimeras resulting from the blastocysts to wild type animals, testing the offspring resulting from these breedings for the presence of the targeted mutation, breeding heterozygous animals, optionally to generate homozygous knock-out animals.

Embryonic stem cells used in the art which may also be used in the methods of this invention comprise for example embryonic stem cells derived from mouse strains such as C57BL/6, BALB/c, DBA/2, CBA/ and SV129. Preferably, embryonic stem cells derived from C57BL/6 mice are used (Seong, E et al (2004) Trends Genet. 20, 59-62; Wolfer, D.P. et al., Trends Neurosci. 25 (2002): 336-340).

The present invention further provides the non-human knock-out animal produced by any of the above described methods.

In another embodiment of the invention, a non-human knock-out animal is provided, whose one or both alleles of *TAAR1* gene is mutated or truncated in a way that less or no active TAAR1 protein is expressed. Preferably, one or both alleles of the *TAAR1* gene of the non-human knock-out animal are replaced with a reporter gene Preferably, the reporter gene is LacZ.

In a preferred embodiment, a non-human knock-out animal is provided whose one or both alleles of a *TAAR1* gene comprise the TAAR1^{NLSlacZ} allele as depicted in Fig 1A. In another preferred embodiment non-human knock-out animal whose one or both alleles of a *TAAR1* gene comprise the construct TAAR1-KO (see Fig. 1B) incorporated in the plasmid pSKDT-Tar1-NLS-PGK-Neo deposited under accession number DSM 17504 (Deposition date: 16.08.2005).

The non-human knock-out animal may be any animal known in the art, which may be used for the methods of the invention. Preferably, the animal of the invention is a mammal, more preferred the knock-out animal of the invention is a rodent. The most preferred non-human knock-out animal is a mouse. Even more preferably, the non-human knock-out animal is a co-isogenic mutant mouse strain of C57BL/6.

The present invention also relates to descendants (= progeny) of the non-human knock-out animals as provided by the invention, obtained by breeding with the same or with another genotype. Descendants may also be obtained by breeding with the same genetic background.

The knock-out animals can be used for preparing primary cell cultures, and for the preparation of secondary cell lines derived from primary cell preparations of these animals. Furthermore, the knock-out animals can be used for the preparation of tissue or organ explants, and cultures thereof. In addition, the knock-out animal maybe used for the preparation of tissue or cell extracts such as membrane or synaptosomal preparations.

The present invention further provides primary cell cultures, as well as secondary cell lines derived from the non-human knock-out animals as provided by the invention or its descendants. In addition, the present invention provides tissue or organ explants and cultures thereof, as well as tissue or cell extracts derived from non-human knock-out animals as provided by the invention or its descendants. Tissue or cell extracts are for example membrane or synaptosomal preparations.

Integration of the genetic construct into the genome can be detected by various methods comprising genomic Southern blot and PCR analysis using DNA isolated e.g. from tail biopsies of the animals.

It will be apparent to the person skilled in the art that there are a large number of analytical procedures which may be used to detect the expression of the reporter gene comprising methods at the RNA level such as for example mRNA quantification by reverse transcriptase polymerase chain reaction (RT-PCR) or by Northern blot, *in situ* hybridization, as well as methods at the protein level comprising histochemistry, immunoblot analysis and *in vitro* binding studies. Quantification of the expression levels of the targeted gene can moreover be determined by the ELISA technology, which is common to those knowledgeable in the art.

Quantitative measurement can be accomplished using many standard assays. For example, transcript levels can be measured using RT-PCR and hybridization methods including RNase protection, Northern blot analysis, and RNA dot blot analysis. Protein levels can be assayed by ELISA, Western blot analysis, and by comparison of immunohistochemically or histochemically stained tissue sections. Immunohistochemical staining, enzymatic histochemical stainings as well as immunoelectron microscopy can also be used to assess the presence or absence of the TAAR1 protein. The TAAR1 expression may also be quantified making use of the NLSlacZ reporter in the TAAR1 non-human knock-out animal using immunohistochemical or histochemicallacZ stainings on tissue sections or quantitative enzymatic lacZ assays performed with tissue homogenates or tissue extracts. Specific examples of such assays are provided below.

The knock-out animals of the invention may be further characterized by methods known in the art, comprising immunohistochemistry, electron microscopy, Magnetic Resonance Imaging (MRI), Positron Emission Tomography (PET) and by behavioral and physiological studies addressing neurological, sensory, and cognitive functions as well as physiologcal (e.g. metabolic) parameters. Examples of behavioral tests and physiological examinations are: Spontaneous behavior, behavior related to cognitive functions, pharmacologically-disrupted behavior, grip strength test, horizontal wire test, forced swim test, rotarod test, locomotor activity test, Prepulse inhibition test, Morris water maze test, Y-maze test, light-dark preference test, passive and active avoidance tests, marble burying test, plus maze test, learned helplessness test, stress-induced hyperthermia, measuring food consumption and development of body weight over time, measuring body temperature and energy consumption under resting and basal conditions and during heat and cold exposure, determining the thermoneutral zone, determining the food assimilation coefficient (e.g. by bomb calorimetry), determining the energy assimilation and the energy content of feces, determining the respiratory coefficient e.g. for analysis of the carbohydrate and lipid metabolism, determining the substrate utilization and energy expenditure during food restriction, determining the oxygen consumption, CO₂- and heat production e.g. by indirect calorimetry, measuring the heart rate and blood pressure under resting, basal and stress conditions (e.g. by telemetry), determining the body composition (e.g. regarding water content, fat amount and fat-free mass).

"Oligonucleotide"and"nucleic acid'refer to single or double-stranded molecules which may be DNA, comprised of the nucleotide bases A, T, C and G, or RNA, comprised of the bases A, U (substitutes for T), C, and G. The oligonucleotide may represent a coding strand or its complement. Oligonucleotide molecules may be identical in sequence to the sequence, which is naturally occurring or may include alternative codons, which encode the same amino acid as that which is found in the naturally occurring sequence (see, Lewin "Genes V" Oxford University Press Chapter 7, 1994, 171-174). Furthermore, oligonucleotide molecules may include codons, which represent conservative substitutions of amino acids as described. The oligonucleotide may represent genomic DNA or cDNA.

The term "allele" as used herein refers to any alternative form of a gene that can occupy a particular chromosomal locus.

The term "promoter" of a gene as used herein refers to the regions of DNA which control the expression of the gene. The TAAR1 promoter is substantially the promoter which controls the expression of the *TAAR1* gene in a wildtype animal. Optionally, the genomic homologous sequences may comprise a part of the TAAR1 promoter or the whole TAAR1 promoter. The homologous sequences may optionally also comprise other TAAR1 regulatory elements.

The term "knock-out animal'as used herein refers to non-human animals comprising a targeted null-mutation of a gene function.

A further objective of the present invention is the use of the non-human knock-out animal as described, or a primary cell culture or secondary cell lines, tissue or organ explants and cultures thereof, or tissue or cell extracts derived from said animals, as a model for identifying and testing for a therapeutic effect of a compound in disorders comprising depression, anxiety disorders, bipolar disorder, attention deficit hyperactivity disorder, stress-related disorders, psychotic disorders such as schizophrenia, neurological diseases such as Parkinson's Disease, neurodegenerative disorders such as Alzheimer's Disease, epilepsy, migraine, hypertension, substance abuse and metabolic disorders such as eating disorders, diabetes, diabetic complications, obesity, dyslipidemia, disorders of energy consumption and assimilation, disorders and malfunction of body temperature homeostasis, disorders of sleep and circadian rhythm, and cardiovascular disorders.

Additionally, these non-human knock-out animals as described above, these cell cultures, cell lines, tissue or organ explants, cultures, or tissue or cell extracts derived from said animals, may be used as a model for studying the TAAR signaling pathway.

Furthermore, these non-human knock-out animals as described above, these cell cultures, cell lines, tissue or organ explant cultures, or tissue or cell extracts derived from said animals, may be used as a tool for assessing TAAR1 function, in particular for assessing the TAAR1 function in disorders such as depression, anxiety disorders, bipolar disorder, attention deficit hyperactivity disorder, stress-related disorders, psychotic disorders such as schizophrenia, neurological diseases such as Parkinson's Disease, neurodegenerative disorders such as Alzheimer's Disease, epilepsy, migraine, hypertension, substance abuse and metabolic disorders such as eating disorders, diabetes, diabetic complications, obesity, dyslipidemia, disorders of energy consumption and assimilation, disorders and malfunction of body temperature homeostasis, disorders of sleep and circadian rhythm, and cardiovascular disorders and disorders involving catecholamine neurotransmitters.

Furthermore, these non-human knock-out animals as described above, these cell cultures, cell lines, tissue or organ explant cultures, or tissue or cell extracts derived from said animals, may also be used as a tool for determining the specificity of compounds acting on TAAR1.

In addition, these non-human knock-out animals as described above, these cell cultures, cell lines, tissue or organ explant cultures, or tissue or cell extracts derived from said animals, may be used as a tool for the identification of so far unknown ligands of TAAR1, and for the characterization of novel ligands acting on TAARs other than TAAR1.

The present invention further provides a method of testing TAAR1 agonists, TAAR1 partial agonists, TAAR1 positive or negative modulators (e.g. TAAR1 enhancer) or TAAR1 inhibitor compounds for effects other than TAAR1-specific effects which method comprises administering a TAAR1 agonist, a TAAR1 partial agonist, a TAAR1 positive or negative modulator (e.g. TAAR1 enhancer) or a TAAR1 inhibitor compound to a non-human knock-out animal as described above, or primary cell culture, or a secondary cell line, or a tissue or organ explant or a culture thereof, or tissue or organ extracts derived from said non-human knock-out animals or its descendants, and determining the effect of the compound comprising assessing neurological, sensory, and cognitive functions as well as physiological (e.g. metabolic) parameters and comparing these to the effect(s) of the same compound on wild type control animals. These neurological, sensory, and cognitive functions and physiological parameters are determined by behavior and physiological studies addressing these functions and parameters.

Control may comprise any animal, primary cell culture, a secondary cell line, a tissue or organ explant or a culture thereof, or tissue or organ extracts, wherein the *TAAR1* gene is not mutated in a way, that less or no active TAAR1 protein is expressed, or wherein the animal, primary cell culture, or a secondary cell line, or a tissue or organ explant or a culture thereof, or tissue or organ extracts comprises the native *TAAR1* gene. Preferably, the control is a wildtype animal.

Furthermore, the use of the non-human knock-out animal as described, or a primary cell culture, or a secondary cell line, or a tissue or organ explant or a culture thereof, or tissue or organ extracts derived from said non-human animal or it descendants is provided for testing of TAAR1 agonists, TAAR1 partial agonists, TAAR1 positive and negative modulators (e.g. TAAR1 enhancer) or TAAR1 inhibitor compounds for effects other than TAAR1-specific effects.

Effects other than TAAR1-specific effects maybe any side-effects of TAAR1 agonists, TAAR1 partial agonists, TAAR1 positive and negative modulators (e.g. TAAR1 enhancer) or TAAR1 inhibitor compounds produced by its interaction with any other molecule.

The term "Agonist" as used herein refers to a compound that binds to and forms a complex with a receptor and elicits a full pharmacological response which is specific to the nature of the receptor involved.

The term "Partial agonist" as used herein refers to a compound that binds to and forms a complex with a receptor and elicits a pharmacological response, which unlike for a full agonist, does not reach the maximal response of the receptor.

The term "Antagonist" as used herein refers to a compound that binds to and forms a complex with a receptor and acts inhibitory on the pharmacological response of the receptor to an agonist or partial agonist. Per definition the antagonist has no influence on receptor signaling in the absence of an agonist of partial agonist for that receptor.

The term"Modulator" as used herein, refers to a compound that binds to and forms a complex with a receptor, and that alters the pharmacological response of the receptor evoked by agonists or partial agonists in a quantitative manner.

The present invention further relates to a test system for testing TAAR1 agonists, TAAR1 partial agonists, TAAR1 positive and negative modulators (e.g. TAAR1 enhancer) or TAAR1 inhibitor compounds for effects other than TAAR1-specific effects comprising a non-human knock-out animal whose one or both alleles of a *TAAR1* gene are mutated and/or truncated in a way that less or no active TAAR1 protein is expressed, or a primary cell culture, or a secondary cell line, or a tissue or organ explant or a culture thereof, or tissue or organ extracts derived from said non-human animal or it descendants, and a means for determining whether TAAR1 agonists, TAAR1 partial agonists, TAAR1 positive and negative modulators (e.g. TAAR1 enhancer) or TAAR1 inhibitor compounds exhibit effects other than TAAR1-specific effects.

In addition, the present invention provides a use of the non-human knock-out animal, whose one or both alleles of a TAAR1 gene are mutated and/or truncated in a way that less or no TAAR1 protein is expressed, or a primary cell culture, or a secondary cell line, or a tissue or organ explant or a culture thereof, or tissue or organ extracts derived from said non-human animal or its descendants for studying the intracellular trafficking of TAARs or of other cellular components linked to TAARs.

Furthermore, the present invention provides a use of the non-human knock-out animal, whose one or both TAAR1 alleles are replaced by a reporter gene for determining the TAAR1 expression profile. The expression profile can be readily analyzed because the reporter gene is expressed with the same spatiotemporal profile in the TAAR1 knock-out as is TAAR1 in wild type animals.

The invention further provides the knock-out animals, methods, compositions, kits, and uses substantially as described herein before especially with reference to the foregoing examples.

Having now generally described this invention, the same will become better understood by reference to the specific examples, which are included herein for purpose of illustration only and are not intended to be limiting unless otherwise specified, in connection with the following figures.

Figures
Figure 1A shows a schematic representation of the TAAR1 wildtype allele TAAR^{+/+} allele (top) with a selection of restriction sites. Arrows represent oligonucleotides which were used for PCR amplification of the 5' arm and 3' arm of the targeting vector (bottom) from genomic DNA. The genomic sequence elements of the wildtype locus which were included into the targeting vector are indicated by dotted lines. The NsiI sites used to clone these genomic arms into the targeting vector are marked in bold lettering.
   The resulting targeting vector pSKDT-Tar1-NLS-lacZ-PGK-Neo (bottom) comprises a genetic construct consisting of a genomic 5' arm (5'arm), the NLS-lacZ reporter-gene, a PGK-Neo resistance-gene and a 3'genomic arm (3'arm). The Diphtheria toxin gene (Dipht.) is placed at the 3'side of the construct.
Figure 1B shows a schematic representation of the TAAR1-KO construct.

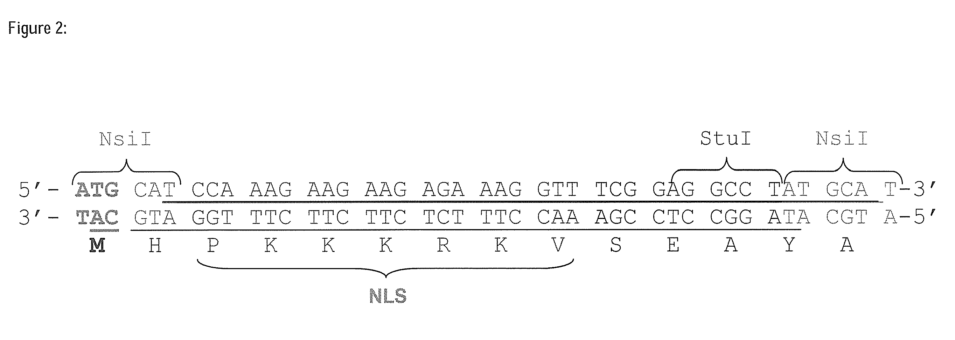

Figure 2 shows the synthetic N-terminal NLS-signal of the lacZ reporter-gene. Oligonucleotides mTar1-29cA (underlined 5'-3'strand) and mTar1-30ncB (underlined 3'-5'strand) inserted in the NsiI sites and resulting amino acid of the NLS (PKKKRKV) sequence in single amino acid letter code. The start codon (ATG) of mTAAR1 is indicated with bold letters. The StuI site used to insert the lacZ-PGK-Neo cassette is indicated in the figure.

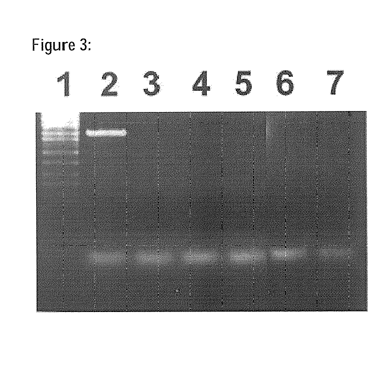

Figure 3 shows a PCR to identify clones with correct targeting at the 3' arm of the NLSlacZ construct. 0.8% agarose gel of PCR reactions with oligonucleotides IL4-neo and mTar26nc on ES-cell clones. Lane 1: DNA molecular weight marker IV (Roche Diagnostics, Mannheim, Germany), Lane 2: clone IIB1, Lane 3: clone IIB2, Lane 4: clone IIB3, Lane 5: clone IIB4, Lane 6: C57BL/6 ES-cell DNA (negative control), Lane 7: water control

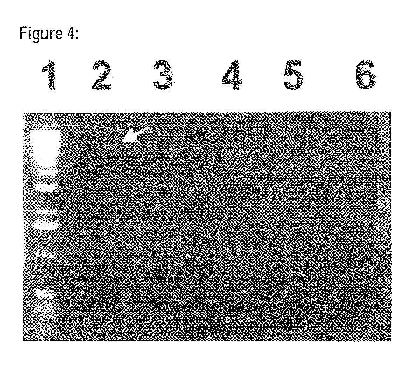

Figure 4 shows a PCR to identify clones with correct targeting at the 5'arm. 0.8% agarose gel of PCR reactions with oligonucleotides IL4-rev and mTar24c on ES-cell clones. Lane 1: DNA molecular weight marker X (Roche Diagnostics, Mannheim, Germany), Lane 2: clone IIB1, Lane 3: clone IIB2, Lane 4: clone IIB3, Lane 5: clone IIB4, Lane 6: C57BL/6 ES-cell DNA (negative control). The arrow indicates the amplification product obtained from clone IIB1.

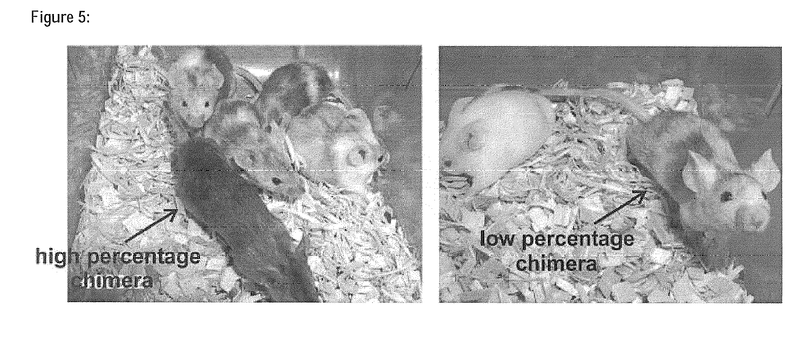

Figure 5 shows mice generated from Balb/c blastocysts and the mutant C57BL/6 mouse embryonic stem cell line as described above. The coat color can be used as an indicator for the degree of chimerism. The amount of black versus white hairs in the fur gives a rough quantitative measure for the degree of the overall chimerism.

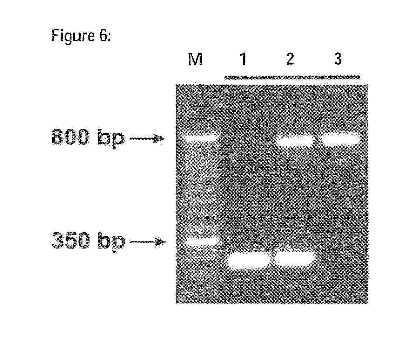

Figure 6 shows a genotype analysis by means of PCR. Genomic DNA was analyzed for the presence of the TAAR1^{+/+} or TAAR1^{NLSlacZ} allele, indicating the respective genotypes of the animals. The 50 bp DNA ladder (Invitrogen) was used as molecular weight standard. M: 50 bp ladder, lane 1: TAAR1^{NLAlacZ/NLSlacz}, lane 2: TAAR1^{+/NLSlacz}, lane 3: TAAR1^{+/+}

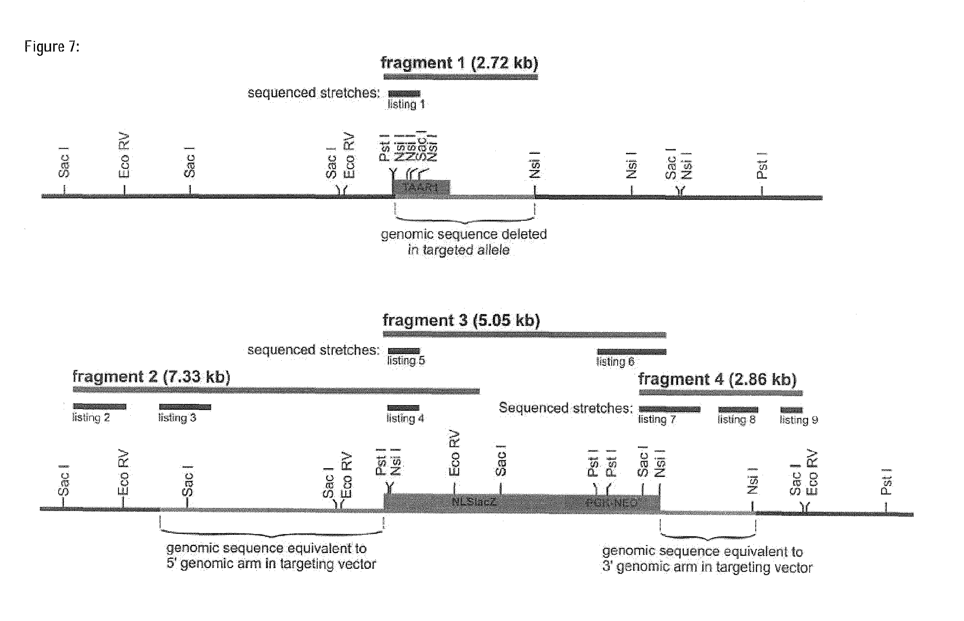

Figure 7 shows a schematic structure of the TAAR1⁺ (top) and TAAR1^{NLSlacZ} allele (bottom). The PCR amplification and partial sequence analysis of the indicated DNA fragments (fragment 1-4; listing 1-9) confirmed the correct homologous recombination of the TAAR1^{NLSlacZ} allele.
   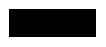 Sequenced stretches (listing 1 to 9)

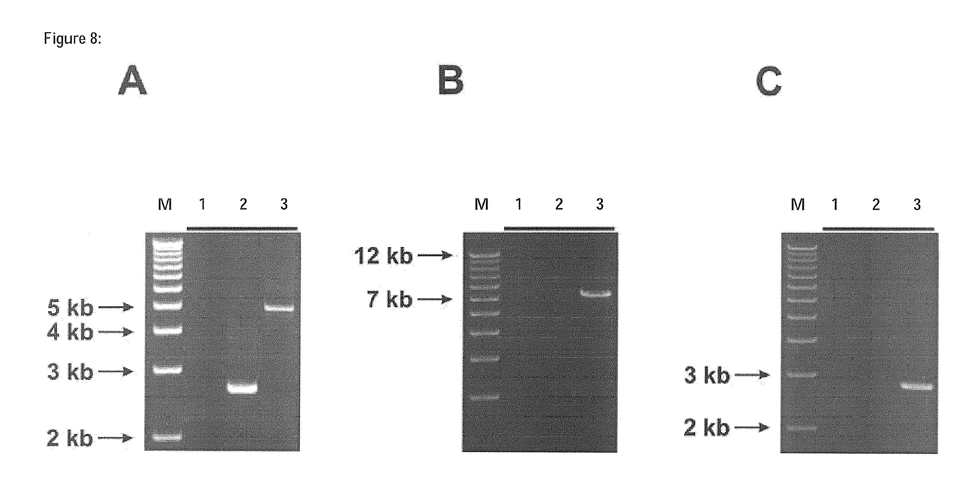

Figure 8 shows an agarose gel of an electrophoresis of PCR amplified DNA fragments as summarized in Table 1.
   A: Under the conditions of PCR protocol 5.1.1 (Table 1), a 2.72 kb PCR product (fragment 1, Fig. 7) was amplified from TAAR1^{+/+} genomic DNA, and a 5.05 kb (fragment 3, Fig. 7) PCR product was amplified from TAAR1^{NLSlacZNLSlacZ} genomic DNA.
      M: 1kb ladder, lane 1: ∅ template (= without template), lane 2: TAAR1^{+/+}, lane 3: TAAR1^{NLSlacZ/NLSlacZ}.
   B: Under the conditions of PCR protocol 5.1.2 (Table 1), a 7.33 kb PCR product (fragment 2, Fig. 7) was amplified from TAAR1^{NLSlacZNLSlacZ} genomic DNA, while no product was obtained from TAAR1^{+/+} genomic DNA.
      M: 1kb ladder, lane 1: Ø template (= without template), lane 2: TAAR1^{+/+}, lane 3: TAAR1^{NLSlacZ/NLSlacZ.}
   C: Under the conditions of PCR protocol 5.1.3 (Table 1), a 2.86 kb PCR product (fragment 4, Fig. 7) was amplified from TAAR1^{NLSlacZNLSlacZ} genomic DNA, while no product was obtained from TAAR1^{+/+} genomic DNA.
      M: 1kb ladder, lane 1: ∅ template (= without template), lane 2: TAAR1^{+/+}, lane 3: TAAR1^{NLSlacZ/NLSlacZ}.
   The 1 kb DNA ladder (Invitrogen) was used as molecular weight standard.

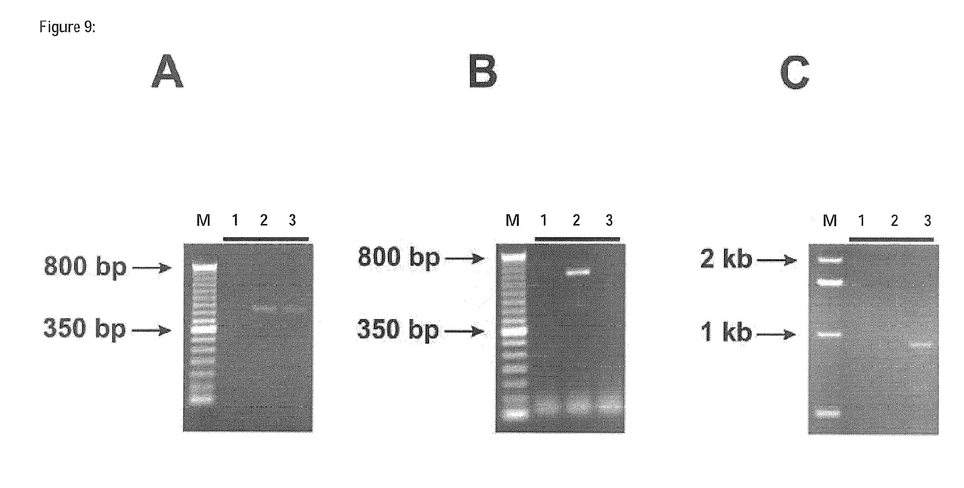

Figure 9 shows an agarose gel of an electrophoresis of PCR products amplified from TAAR1^{+/+} and TAAR1^{NLSlacZ/NLSlacZ} mouse brain cDNA preparations, respectively, as summarized in Table 2.
   A: PCR reactions specific for GAPDH (see above) on TAAR1^{+/+} and TAAR1^{NLSlacZ/NLSlacZ} mouse brain cDNA preparations. From both cDNAs a 452 bp PCR product was amplified.
      M: 50bp ladder, lane 1: ∅ template (= without template), lane 2: TAAR1^{NLSlacZ/NLSlacZ}, lane 3: TAAR1^{+/+}.
   B: PCR reactions specific for NLSlacZ (see above) on TAAR1^{+/+} and TAAR1^{NLSlacZ/NLSlacZ} mouse brain cDNA preparations. A 631 bp PCR product was amplified from TAAR1^{NLSlacZ/NLSlacZ}, but not from TAAR1^{+/+} mouse brain cDNA.
      M: 50bp ladder, lane 1: ∅ template (= without template), lane 2: TAAR1^{NLSlacZ/NLSlacZ}, lane 3: TAAR1^{+/+}.
   C: PCR reactions specific for TAAR1 (see above) on TAAR1^{+/+} and TAAR1^{NLSlacZ/NLSlacZ} mouse brain cDNA preparations. A 936 bp PCR product was amplified from TAAR1^{+/+}, but not from TAAR1^{NLSlacZ/NLSlacZ} mouse brain cDNA.
      M: 1 kb ladder, lane 1: ∅ template (= without template), lane 2: TAAR1^{NLSlacZ/NLSlacZ}, lane 3: TAAR1^{+/+}.
   The 50 bp DNA ladder (Invitrogen; A, B) and the1 kb DNA ladder (Invitrogen; C) were used as molecular weight standards.

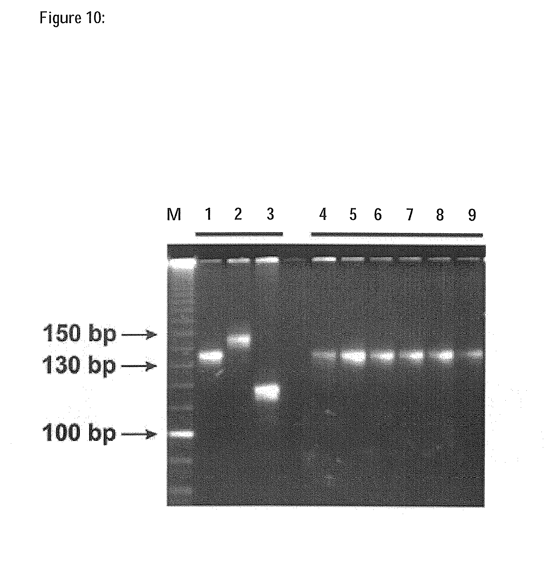

Figure 10 shows an agarose gel of an electrophoresis of PCR products from the microsatellite analysis of 5 TAAR1^{+/NLSlacZ} mice of the F1 generation, the ES cell line used for generation of germline chimeras and samples of the mouse inbred strains C57BL/6, DBA and SV129 (result for the microsatellite marker D5MIT259). The match of the standard sample for C57BL/6 with all test samples provides evidence that the mutant mouse line carrying the TAAR1^{+/NLSlacZ} allele is on a C57BL/6 genetic background.
   The 10 bp DNA ladder (Invitrogen) was used as molecular weight standard.
   M: 10bp ladder, lane 1: C57BL/6, lane 2: DBA, lane 3:SV129, lane 4: ES TAAR1^{+/NLSlacZ}, lane 5: F1 #1 TAAR1^{+/NLSlacZ}, lane 6: F1 #2 TAAR1^{+/NLSlacZ}, lane 7: F1 #3 TAAR1^{+/NLSlacZ}, lane 8: F1 #4 TAAR1^{+/NLSlacZ}, lane 9: F1 #5 TAAR1^{+/NLSlacZ}.

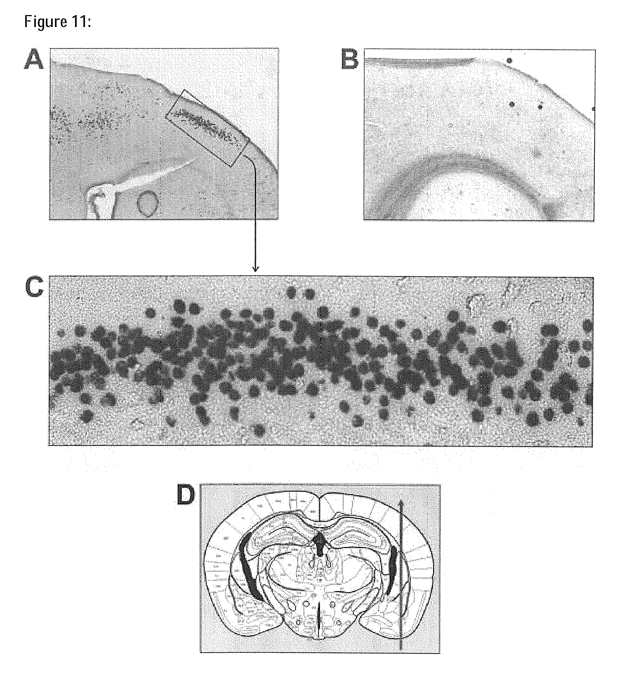

Figure 11 shows a LacZ staining of histological sections of adult TAAR1^{NLSlacZ/NLSlacZ} and TAAR1^{+/+} mouse brains. (A)The TAAR1^{NLSlacZ/NLSlacZ} mouse brain section displays a strong, specific staining; (B): staining is absent from the TAAR1^{+/+} mouse brain section , (C): higher magnification of boxed area in (A). Both sections were cut in sagittal orientation from equivalent brain regions (see D for schematic diagram of the brain regions from which the sections were cut).

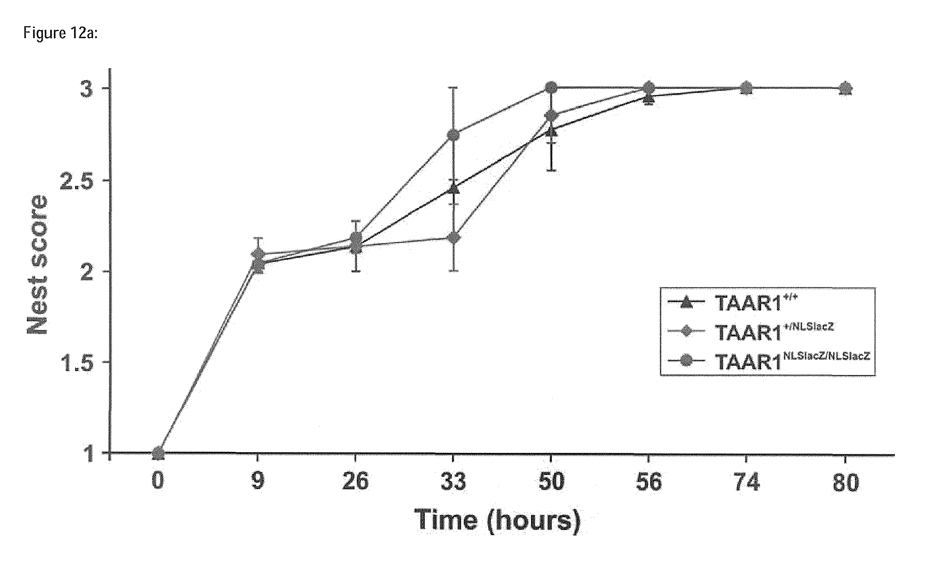

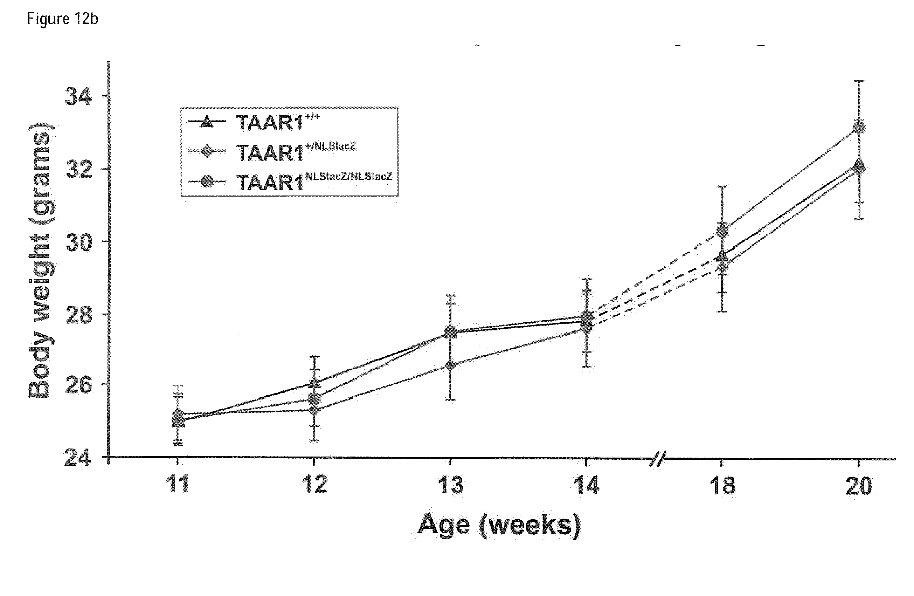

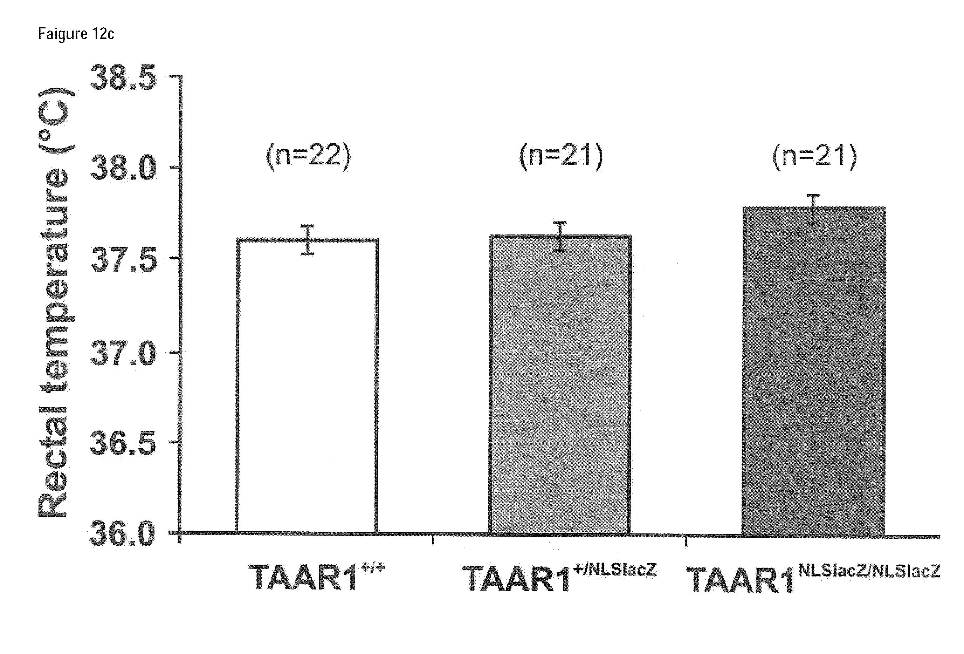

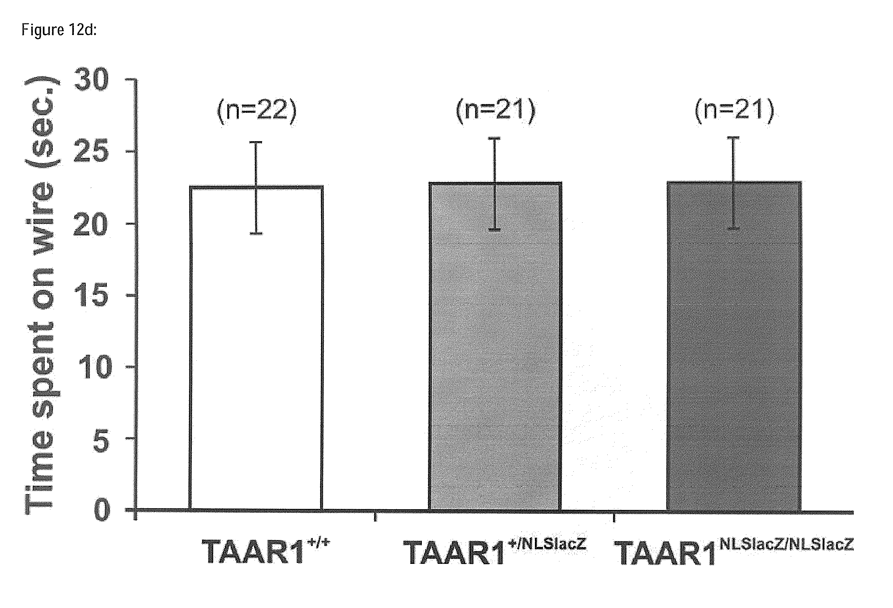

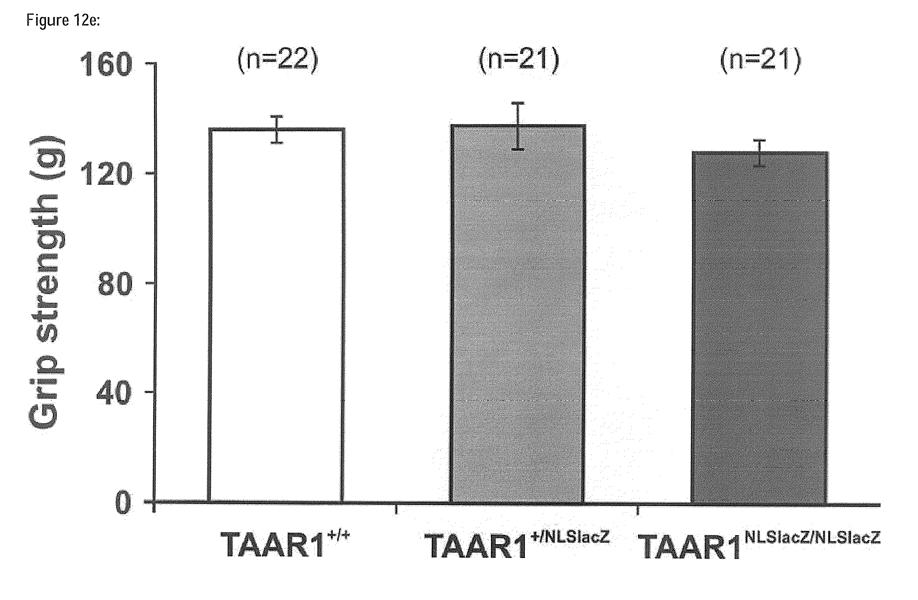

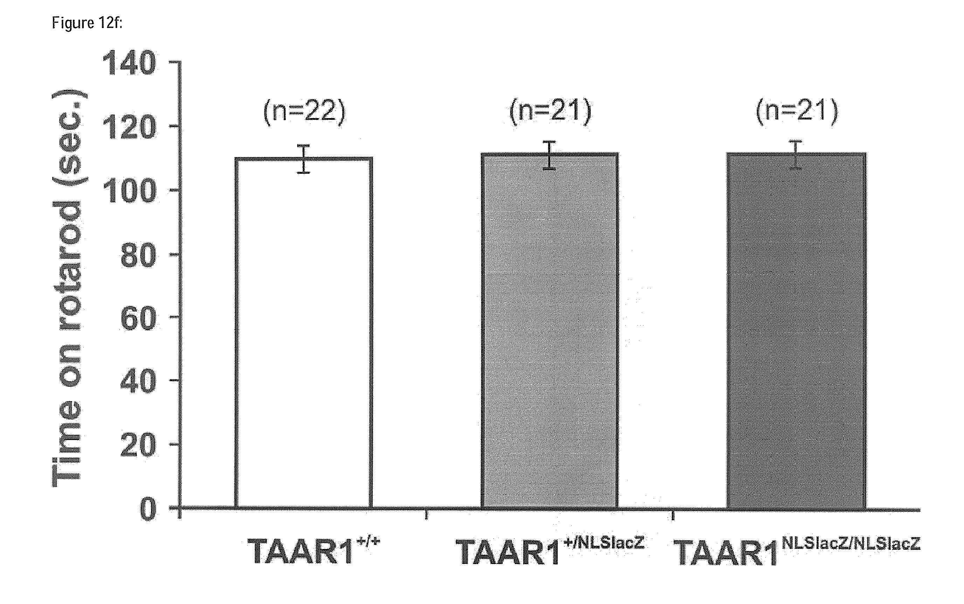

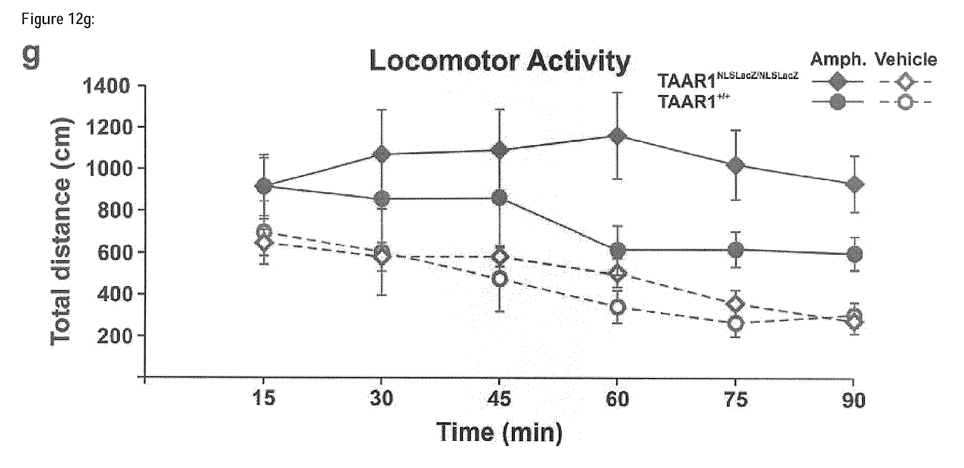

Figure 12 show graphical representation of physical properties of the TAAR1^{LacZ} mouse mutant. Nest building behaviour (a) and development of body weight (b) of TAAR1^{+/+} (n = 22), TAAR1^{+/LacZ} (n = 21) and TAAR1^{LacZ/LacZ} (n = 21) mice showed no differences between the genotypes. Rectal body temperature (c) of TAAR1^{+/+} (n = 22), TAAR1^{+/LacZ} (n = 21) and TAAR1^{LacZ/LacZ} (n = 21) mice. No statistical significant difference in body temperature between genotypes was observed. Assessment of the physical strength of TAAR1^{+/+} (n = 22), TAAR1^{+/LacZ} (n = 21) and TAAR1^{LacZ/LacZ} (n = 21) mice by means of the horizontal wire test (d) and grip strength (e) did not reveal any significant differences between genotypes. Motor coordination and balance revealed by the performance on the rotarod (f). No significant differences between the genotypes have been observed. (g) Locomotor activity of TAAR1^{+/+} and TAAR1^{LacZ/LacZ} mice after a single application of d-amphetamine (2.5 mg/kg i.p., n=12). The increase in locomotor activity triggered by the amphetamine challenge was significantly higher in TAAR1^{LacZ/LacZ} mice as compared to their wild type littermates (filled symbols, straight lines) while there were no significant differences between genotypes in vehicle treated animals (open symbols, dashed lines).

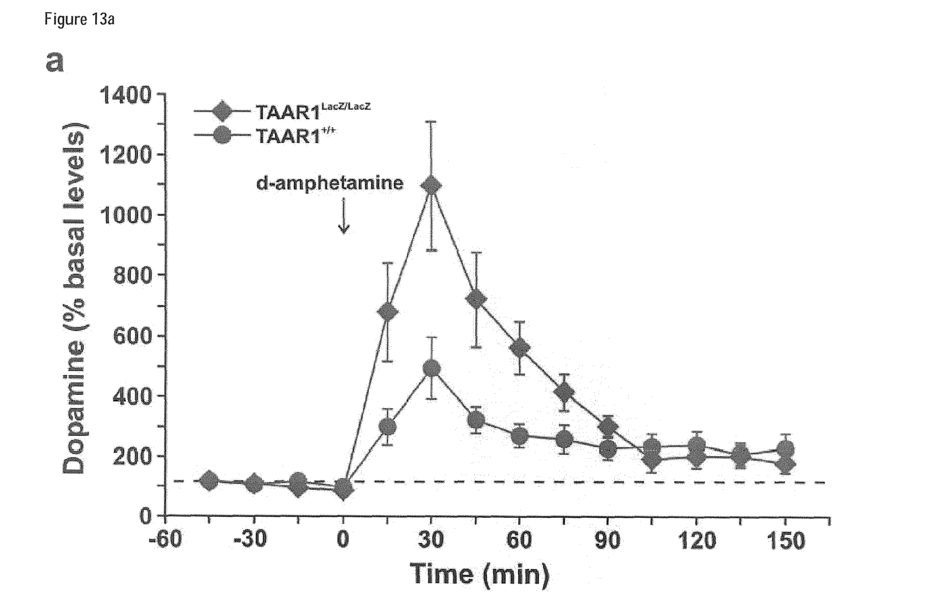

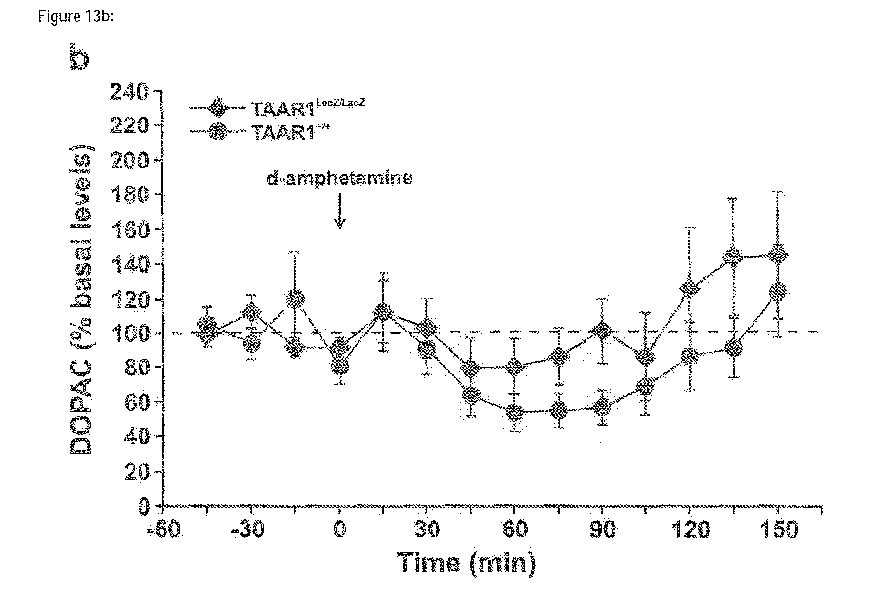

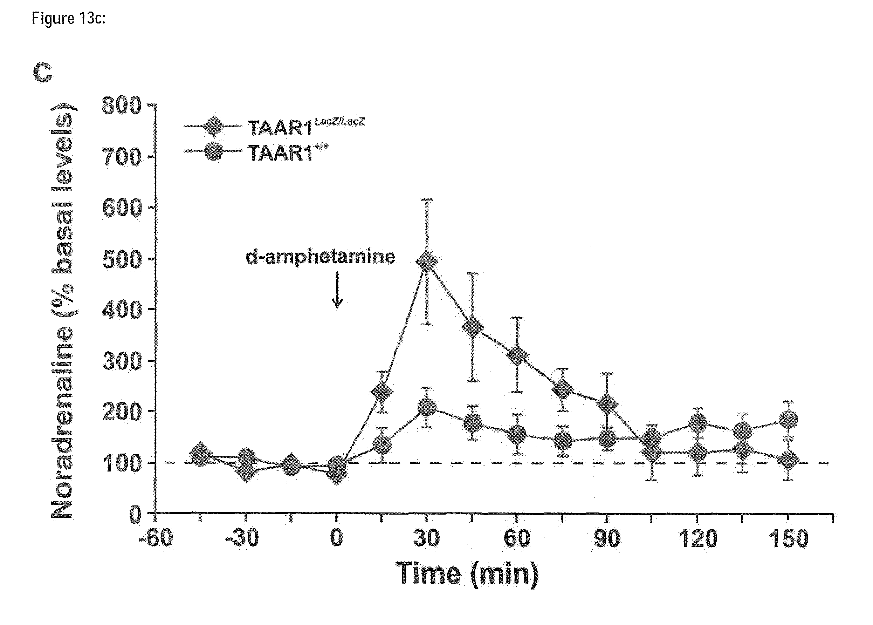

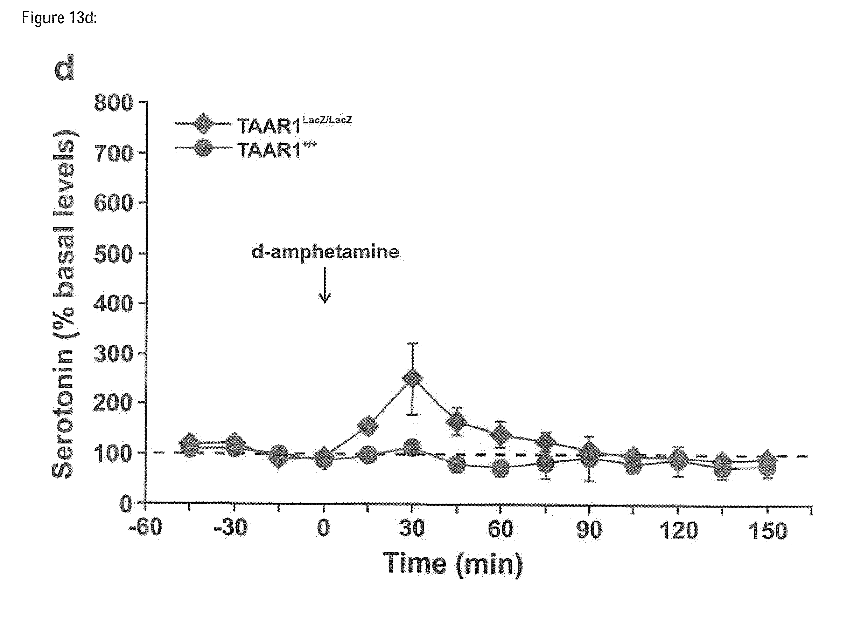

Figure 13 shows a graphical representation of increased amphetamine-triggered transmitter release in the striatum in absence of TAAR1 revealed by *in vivo* microdioalysis. Extracellular levels of dopamine, 3,4-dihydroxyphenylacetic acid (DOPAC), noradrenaline and serotonin in the striatum of TAAR1^{+/+} and TAAR1^{LacZ/LacZ} mice after a single application of d-amphetamine (2.5 mg/kg i.p., n=7-8) as revealed by *in vivo* microdialysis. Dialysates of the same animals were analyzed for all four compounds. (a-b) The amphetamine-triggered increase in the extracellular dopamine levels is 2.3 fold as big in the striatum of TAAR1^{LacZ/LacZ} mice as in their TAAR1^{+/+} littermates, while there is only a marginal decrease in the levels of the dopamine catabolite DOPAC in both TAAR1^{LacZ/LacZ} and TAAR1^{+/+} animals in response to amphetamine with no significant differences between the genotypes. (c) The increase in the level of noradrenalin in response to the amphetamine challenge is 2.4-fold as big as in wild type animals. (d) A 2.5-fold increase in the serotonin level triggered by amphetamine was observed only in TAAR1^{LacZ/LacZ} mice, but not in their TAAR1^{+/+} littermates.

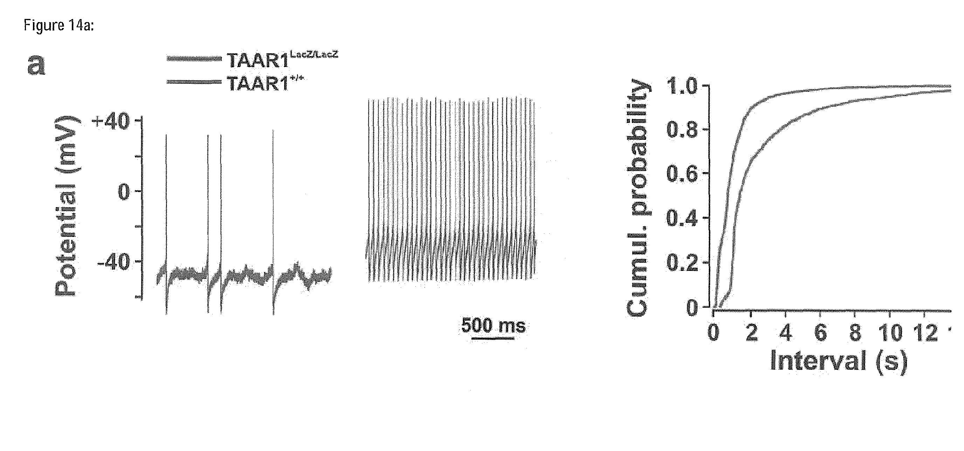

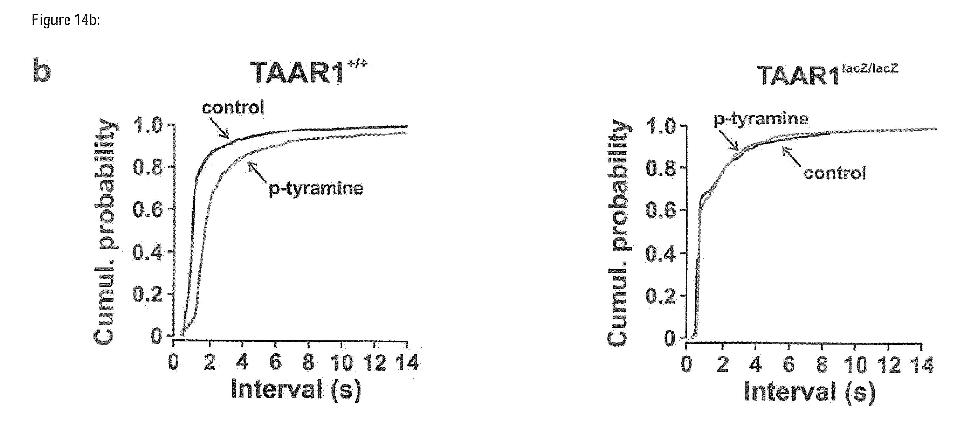

Figure 14 shows a graphical representation of electrophysiological analysis of dopaminergic neurons in the VTA of TAAR1^{LacZ/LacZ} and wild type mice. (a) The spontaneous firing rate of dopaminergic neurons is lower in the wild type (left panel) than in the TAAR1^{LacZ/LacZ} mice (right). Cumulative probability histogram of spike intervals in the wild type (black trace) and TAAR1^{LacZ/LacZ} mice (gray). In the TAAR1^{LacZ/LacZ} mice, the distribution of interevent intervals is significantly shifted to the left, indicating an increase in the spontaneous spike frequency. (b) The TAAR1 agonist p-tyramine decreases the firing rate of dopaminergic neurons in the wild type but not in the TAAR1^{LacZ/LacZ} mice, as shown by the shift in the cumulative probability histogram of interevent intervals in the wild type (left) but not in the TAAR1^{LacZ/LacZ} mice (right).

### Examples

Commercially available reagents referred to in the examples were used according to manufacturer's instructions unless otherwise indicated.

### Example 1:

### 1) Strategy for gene replacement of the TAAR1 coding sequence by a synthetic NLS-lacZ coding sequence

To target TAAR1 in mouse embryonic stem cells (ES-cells) a gene-targeting vector was constructed and used to generate C57BL/6 mice with a deficiency of TAAR1. The gene-targeting vector completely replaces the TAAR1 coding region as well as about 1,5 kb genomic sequence downstream of the coding sequence with a synthetic NLS-lacZ-PGK-Neo cassette.

It is a replacement type gene targeting vector allowing for positive selection of homogenously recombined ES-cells using the Neomycin-phosphotransferase-gene (Neo) expressed under the control of the phosphoglycerate kinase promoter (PGK) (Galceran J, Miyashita-Lin E.M., Devaney E, Rubenstein J.L.R., Grosschedl R., Development 127 (2000): 469-482). To permit negative selection against ES clones carrying the targeting vector randomly integrated into the genome a diphtheria-toxin gene has been inserted into the vector outside of the TAAR1 genomic sequence (as described in Gabernet L., Pauly-Evers M., Schwerdel C., Lentz M., Bluethmann H., Vogt K., Alberati D., Mohler H., Boison D. Neurosci Lett. 373 (2005): 79-84).

At the same time the lacZ reporter-gene (Galceran J, Miyashita-Lin E.M., Devaney E, Rubenstein J.L.R., Grosschedl R., Development 127 (2000): 469-482) was fused to a nuclear signal sequence (NLS) and placed under the transcriptional control of the putative TAAR1 promoter and regulatory elements. Hereby the start-codon of the synthetic reporter is identical to the start-codon of TAAR1. This allows the sensitive analysis of the expression pattern conferred by the endogenous TAAR1 control region in histochemical stainings for the product of the lacZ gene.

### 2) Cloning of a plasmid for targeting of the NLS-lacZ coding sequence to the TAAR1 gene by homologous recombination in ES-cells

### 2.1) Construction of the TAAR1 gene targeting vector

The resulting targeting vector consists of a genomic 5' arm (5'arm), the NLS-lacZ reporter-gene, a PGK-Neo resistance cassette and a 3' genomic arm. The Diphtheria toxin cassette (Dipht.) is placed at the 3'side of the targeting vector (see Fig. 1).

Oligonucleotides were designed based on the published genomic sequences of the mouse TAAR1 locus (Mouse genome sequence database, NCBI draft 34, May 2005) and obtained from a commercial supplier (Microsynth AG, Balgach, Switzerland). All molecular cloning techniques were carried out essentially according to Sambrook et. al. (Molecular Cloning: A laboratory manual. 1989, Cold Spring Harbor Laboratory Press, Cold Spring Harbor.) and the instructions of the suppliers of kits and enzymes.

The targeting vector pSKDT-Tar1-NLS-lacZ-PGK-Neo contains 4,0 kb genomic sequence 5' of the mouse TAAR1 coding sequence and 1,7kb genomic sequence 3' of the mouse TAAR1 coding sequence (Fig. 1).

These sequences were amplified from genomic C57BL/6 DNA using proofreading PCR and cloned into cloning vectors.

To clone the 5' arm oligonucleotide mTar1- 5'-KpnI-16c and oligonucleotide mTar1-755-nc were used in the following PCR reaction.

2 ng/µl genomic C57BL/6 DNA, 200 µM dNTPs (PCR Nucleotide Mix, Roche Diagnostics, Mannheim, Germany), 0,5 µM of each oligonucleotide, 1x PCR buffer (Promega, Madison, WI, USA), 0,06 U/µl Pfu Polymerase (Promega, Madison, WI, USA) in a total volume of 100 µl were incubated with the following protocol: 95°C 2 min., 35x (95°C 45 sec., 59°C 45 sec., 72°C 9 min.), 72°C 7 min, ∞ 4°C on a PCR Thermocycler MJ Research PTC-200 (MJ Research Inc., Watertown, USA). The resulting PCR product of 4,702kb contained the 5' arm of the TAAR1 locus and was cloned into the SrfI site of pPCR-Script Amp SK+ (Invitrogen-Cribco, Carlsbad, CA, USA).

Orientation and parts of the sequence were confirmed by sequencing using the BigDye Terminator v1.1 Cycle Sequencing Kit (Applied Biosystems) and an ABIPrism 310 Genetic Analyzer.

The resulting vector is pPCR-Script-5'Tar1.

To clone the 3' arm oligonucleotide mTar1-33c and oligonucleotide mTar1-SmaI-1lnc were used in the following PCR reaction.

2ng/µl genomic C57BL/6 DNA, 200 µM dNTPs (PCR Nucleotide Mix, Roche Diagnostics, Mannheim, Germany), 0,5 µM of each oligonucleotide, 1x PCR buffer (Promega, Madison, WI,USA), 0,06 U/µl Pfu Polymerase (Promega, Madison, WI,USA) in a total volume of 100 µl were incubated with the following protocol: 95°C 2 min., 35× (95°C 45 sec., 60°C 45 sec., 72°C 4 min.), 72°C 7 min, ∞ 4°C on a PCR Thermocycler MJ Research PTC-200 (MJ Research Inc., Watertown, USA).

The resulting PCR product of 1651kb was cloned into the SrfI site of pPCR-Script Amp SK+ (Invitrogen-Cribco, Carlsbad, CA, USA).

Orientation and parts of the sequence were confirmed by sequencing using the BigDye Terminator v1.1 Cycle Sequencing Kit (Applied Biosystems) and an ABIPrism 310 Genetic Analyzer.

The resulting vector is pPCR-Script-3'Tar1.

The targeting vector was assembled in 3 steps.

Step 1: The 3' genomic arm and 5' genomic arm cloned as described above were assembled into the plasmid backbone pSK (Stratagene, La Jolla, CA, USA) which contains a diphtheria toxin cassette as described in Gabernet et al. (Enhancement of the NMDA receptor function by reduction of glycine transporter-1 expression. Neurosci Lett. 373 (2005): 79-84). To this end, the 3' genomic arm was removed from the plasmid pPCR-Script-3'Tar1 by restriction digest with ClaI and NotI, and the resulting 1.7 kb genomic fragment was purified by agarose gel electrophoresis and gel extraction. Thereafter, the 1.7 kb genomic DNA fragment was ligated into the plasmid pSK, which previously had been digested with ClaI and NotI. The resulting plasmid was called pSKDT-3'Tar1.

Subsequently, the 5' genomic arm cloned as described above was removed from the plasmid pPCR-Script-5'Tar1 by restriction digest with NsiI and Kpnl and subsequent agarose gel electrophoresis and gel extraction. Following, the 4 kb genomic DNA fragment was ligated into the plasmid pSKDT-3Tar1, which previously had been digested with NsiI and KpnI, resulting in the plasmid pSKDT-5'-3Tar1

Step2: A synthetic sequence harboring several restriction sites (see Fig. 2) as well as a NLS sequence was inserted into the plasmid pSKDT-5'-3'Tar1. To this end, the 5' phosphorylated oligonucleotides mTar1-29cA and mTar1-29cB were annealed. Plasmid pSKDT-5'-3'Tar1 was digested with NsiI, and the annealed oligonucleotides were ligated into this plasmid, resulting in the plasmid pSKDT-5'-3'Tar1-NLS.

Step3: The NLS-lacZ-PGK-Neo cassette was inserted into the plasmid pSKDT-5'-3'Tar1-NLS. For this purpose plasmid pSKDT-5'-3'Tar1-NLS was linearized with a StuI restriction digest. The NLS-lacZ-PGK-Neo cassette was isolated from the plasmid C8βgal (Galceran J, Miyashita-Lin E.M., Devaney E, Rubenstein J.L.R., Grosschedl R. Hippocampus development and generation of den date gyrus granule cells is regulated by LEF1. Development 127 (2000): 469-482) with a Smal restriction digest and subsequent agarose gel electrophoresis and gel extraction. The NLS-lacZ-PGK-Neo cassette DNA fragment was ligated into the StuI linearized plasmid pSKDT-5'-3'Tar1-NLS, resulting in the targeting vector pSKDT-Tar1-NLS-lacZ-PGK-Neo.

Deposition data: The plasmid pSKDT-Tar1-NLS-lacZ-PGK-Neo comprising the genetic construct TAAR1-KO (see Figure 1B) was deposited under the Budapest Treaty at the Deutsche Sammlung von Microorganismen und Zellkulturen GmbH (DSMZ), Mascheroder Weg 1 b, D-38124 Braunschweig, Germany, with an effective deposition date of 16.08.2005 under the accession number DSM 17504.

### 2.2) Gene targeting of the TAAR1 gene in mouse ES cells by homologous recombination

### 2.2.1) Culturing of C57BU6 ES-cells

Handling of ES-cells was performed essentially as described in Joyner (Gene Targeting. 1999, Second Edition, The Practical Approach Series, Oxford University Press, New York). C57BL/6 ES-cells (Eurogentec, Seraing, Belgium) were grown on monolayers of mitotically inactivated primary mouse embryonic fibroblast (MEF) cells isolated from a mouse line CD1-Tg.neoR expressing the neomycin resistance gene (Stewart C.L., Schuetze S., Vanek M., Wagner E.F. Expression of retroviral vectors in transgenic mice obtained by embryo infection. EMBO J. 6 (1987): 383-8). MEFs were isolated as described in (Joyner, AL, eds.: Gene Targeting. A Practical Approach. 2000, Oxford University Press, New York) and mitotically inactivated by gamma radiation (18Sv in a Cs-137 irradiation source).

ES-cells were grown in ES-medium containing Dulbeccos's modified Eagle Medium (Invitrogen-Gibco, Carlsbad, CA, USA) supplemented with 15% FCS (Inotech/Biological Industries, Beit Haemek, Israel), 100 IU/ml Penecillin/Streptomycin (Invitrogen-Gibco, Carlsbad, CA, USA), 0.5 mM β-Mercaptoethanol (Invitrogen-Gibco, Carlsbad, CA, USA), non essential amino acids MEM (1x, Invitrogen-Cibco, Carlsbad, CA, USA), 2 mM Glutamine (Invitrogen-Cibco, Carlsbad, CA, USA) and 1000 U/ml leukocyte inhibitory factor (Chemicon, Temecula, CA, USA).

### 2.2.2) Electroporation of the targeting vector into ES-cells

The SacII linearized targeting vector pSKDT-Tarl-NLS-lacZ-PGK-Neo (total amount: 30µg) was added to 30x10⁶ ES-cells in a buffer containing 137 mM NaCl, 2.7 mM KCl, 90 mM Na₂HPO₄, 1,5 mM KH₂PO₄, pH 7.4 (PBS) and electroporated with a Bio-Rad Genepulzer with a capacity extender (Bio-Rad, Hercules, CA, USA; settings: 280 V, 500µF). Thereafter, ES-cells were plated on MEF mono cell layers and selected for the presence of the Neomycin gene in ES-medium supplemented with 350µg/ml G418 (geneticin, Sigma-Aldrich, St. Louis, MO, USA).

Individual, well separated ES-clones originating from the transfected cells were transferred into 48 well culture dishes and grown for 10 days. 1/3 of the cells were used to isolate genomic DNA using the MagNAPure LC system (Roche Diagnostics, Basel, Switzerland; Laird, P.W., Zijderveld, A., Linders, K., Rudnicki, M.A., Jaenisch, R., Berns, A.: Simplified mammalian DNA isolation procedure. Nucleic Acids Res. 1991; 19 (15): 4293) and analyzed using PCR. The remaining 2/3 of the cells were eventually used for further analysis and for blastocyst injections.

### 2.2.3) Screening of monoclonal ES-cells with PCR for correct targeting of TAAR1

In 62 clones the correct gene targeting event was assessed by PCR amplification of DNA fragments spanning the transition points between the genomic DNA included into the targeting vector and surrounding genomic sequence.

To test for correct recombination at the 3'arm PCR clones were screened with PCRs using oligonucleotides IL4-neo and mTar26nc in the following protocol:

20-100 ng genomic DNA, 1× concentrated PCR buffer 3 (part of Expand High Fidelity PCR System; Roche Diagnostics, Mannheim, Germany), 500 µM of each dNTP (PCR Nucleotide Mix, Roche Diagnostics, Mannheim, Germany), 500 nM of each oligonucleotide (Microsynth AG) and 3.75 U/reaction Expand High Fidelity Enzyme Mix (part of Expand High Fidelity PCR System; Roche Diagnostics). The PCR reactions were run with the following temperature protocol:
94°C 2min., 33× (94°C 15 sec., 62°C 30 sec., 68°C 3,5 min.), 68°C 20 min, ∞ 12°C

This PCR yields PCR products only in the presence of genomic DNA of ES-clones, in which the correct homologous recombination event between the 3' arm of the targeting vector and the chromosomal DNA as depicted in Fig. 1 has occurred. As negative control wildtype DNA (Fig. 3, Lane 6) as well as several ES-clones in which no homologous recombination has occurred (Fig. 3, Lane 3-5) were included into the analysis.

Importantly clone IIB1 gives amplification at the expected size of 3.2kb (Fig. 3., Lane 2).

To test for correct recombination at the 5'arm PCR clones were screened with PCRs using oligonucleotides IL4-rev and mTar24c in the following protocol:
20-100 ng genomic DNA, 1× concentrated PCR buffer 3 (part of Expand High Fidelity PCR System; Roche Diagnostics, Mannheim, Germany), 500 µM of each dNTP (PCR nucleotide Mix, Roche Diagnostics, Mannheim, Germany), 500 nM of each oligonucleotide (Microsynth AG) and 3.75 U/reaction Expand High Fidelity Enzyme Mix (part of Expand High Fidelity PCR System; Roche Diagnostics). The PCR reactions were run with the following temperature protocol:
94°C 2min., 33× (94°C 15 sec., 64°C 30 sec., 68°C 12 min.), 68°C 20 min, ∞ 12°C

This PCR yields PCR products only in the presence of genomic DNA of ES clones, in which the correct homologous recombination event between the 5' arm of the targeting vector and chromosomal DNA as depicted in Fig. 1 has occurred. As negative control wildtype DNA (Fig. 4, Lane 6) as well as several ES clones in which no homologous recombination has occurred (Fig. 4, Lane 3-5) were included into the analysis.

The above PCR conducted with genomic DNA of Clone IIB1 gives an amplification at the expected size of 9.8kb (Fig. 4, Lane 2, arrow).

Clone IIB1 was chosen for injection into blastocysts as described in chapter 3.

Following Oligonucleotides (all sequences in 5'→3' orientation) were used:

| Name | Sequence | SEQ. ID NO: |
|---|---|---|
| mTar1-5'-KpnI-16c | CGGGTACCTGTCACTCACCGGCATTCGG | 10 |
| mTar1-755-nc | CCTTGCTTGTCCTTTAGCTATG | 11 |
| mTar-33c | CCCATGTGACCAATTTGTTCACC | 12 |
| mTar1-3'-SmaI-11nc | CTGCTGCCCGGGATTCAAGCTCTTCTTGACTCTGG | 13 |
| mTar1-29cA | TCCAAAGAAGAAGAGAAAGGTTTCGGAGGCCTATGCA | 14 |
| mTar1-30ncB | TTGGCCTCCGAAACCTTTCTCTTCTTCTTTGGATGCA | 15 |
| IL4-neo | GCGCATCGCCTTCTATCGCC | 16 |
| mTar1-26nc | GAGCTTCACACATGAACACACC | 17 |
| mTar1-24c | GTGGGCTAAGATCTAGGAACG | 18 |
| IL4-rev | GGCGATAGAAGGCGATGCGC | 19 |

### 3) Generation of a TAAR1 - NLSlacZ knock-in mouse line from a mutant ES cell line

All handling of animals was carried out in compliance with Swiss Federal and Cantonal laws on animal research, and permission for the generation and handling of the mutant mouse line was specifically granted from the Kantonale Veterinäramt of Basel City with the Tierversuchgenehmigung No. 2055 as of August 23rd 2004.

A mutant mouse line which carries an inheritable targeted mutation of the TAAR1 gene as described in chapter 1) in all cells was generated following standard procedures essentially as described in Hogan et al. (Manipulating the mouse embryo. 1994, Second Edition, Cold Spring Harbor Press, Cold Spring Harbor.) by Polygene AG (Rümlang, Switzerland). For the generation of chimeric animals, the monoclonal mutant ES cells (clone IIB1, as described in chapter 2) were injected into embryonic day 3.5-4.5 (E3.5-4.5) Balb/c blastocysts and transferred into pseudo-pregnant C57BL/6 x CBA F1 females. Offspring delivered by these females were judged for the degree of chimerism (i.e. the degree of overall contribution of ES cells to the chimeric animals). The employed breeding paradigm allowed to use the coat color as parameter for estimating the degree of chimerism, with the percentage of black hairs in the fur reflecting the degree of chimerism.

Male high percentage chimeras were naturally mated with C57BL/6 females, and all offspring with purely black coat color was analyzed for the presence of the TAAR1^{NLSlacZ} allele. To this end, genomic DNA was isolated from tail biopsies of adult animals with a MagNAPure LC system for nucleic acid purification (Roche Applied Science, Rotkreuz, Switzerland) according to the instructions of the manufacturer and analyzed for the presence of the TAAR1 wild type allele (TAAR1⁺; indicating the presence of the undisrupted TAAR1 gene) as well as the neomycin phosphotransferase coding sequence (indicating the presence of the TAAR1^{NLSlacZ} allele) by means of PCR.

PCR reactions were performed on a GenAmp 9700 thermocycler (Applied Biosystems, Rotkreuz, Switzerland) in a total volume of 50µl per reaction composed as follows (final concentrations/amounts):

20 - 100 ng genomic DNA, 20 mM Tris-HCl (pH8.4), 50 mM KC1, 1.5 mM MgCl2, 200 nM of each oligonucleotide (Microsynth AG, Balgach, Switzerland), 200 mM of each dNTP (Amersham), 5 U/reaction recombinant Taq DNA polymerase (Invitrogen). The targeted or undisrupted TAAR1 alleles were detected simultaneously in the same PCR reaction by the following oligonucleotides:
TAAR1⁺ allele:
TAAR1 31c: 5'-gaaggtggaattctaacctgac-3' (SEQ. ID NO: 20)
TAAR1 D8: 5'-ccttgcttgtcctttagctatg-3' (SEQ. ID NO: 21)
TAAR1^{NLSlacZ} allele:
NEO U1: 5'-cttgggtggagaggctattc-3' (SEQ. ID NO: 22)
Neo D1: 5'-aggtgagatgacaggagatc-3'(SEQ. ID NO: 23)

The PCR reactions were run with the following temperature profile: 95°C 2min., 35x (95°C 30 sec., 57°C 30 sec., 72°C 1 min.), 72°C 5 min, ∞ 4°C. The PCR products were analyzed by standard agarose gel electrophoresis as described in Sambrook et al. (Molecular Cloning: A laboratory manual. 1989, Cold Spring Harbor Laboratory Press, Cold Spring Harbor.), and the expected DNA fragment sizes were 755 bp for the undisrupted TAAR1 allele and 280 bp for the TAAR1^{NLSlacZ} allele.

### 4) Characterization of the TAAR1 - NLSlacZ knock-out

### 4.1) Analysis of the TAAR1 - NLSlacZ gene replacement inherited in the TAAR1 - NLSlacZ knock-out mouse line

In order to proof that the TAAR1^{NLSlacZ} allele equals the designed mutant gene as described in 2 the targeted gene locus was analyzed based on genomic DNA derived from homozygous mutants of the F2 generation. The correct gene targeting event was verified by PCR amplification and DNA sequence analysis of DNA fragments spanning the transition points between the genomic DNA included into the targeting vector and surrounding genomic sequence (see Figure 7).

The DNA fragments 1-4 (see Fig. 7) were amplified by PCR from genomic DNA as template. The genomic DNA was extracted with a MagNAPure LC system for nucleic acid purification (Roche Diagnostics, Basel, Switzerland) from tail biopsies of adult animals carrying either two TAAR1⁺ alleles (genotype: TAAR1^{+/+}) or two TAAR1^{NLSlacZ} alleles (genotype: TAAR1^{NLSlacZ/NLSlacZ}). PCR reactions were performed on a GenAmp 9700 thermocycler (Applied Biosystems, Rotkreuz, Switzerland) in a total volume of 50µl per reaction composed as follows (final concentrations/amounts):

20 - 100 ng genomic DNA, 1× concentrated PCR buffer 3 (part of Expand High Fidelity PCR System; Roche Diagnostics, Mannheim, Germany), 500 µM of each dNTP (Amersham), 300 nM of each oligonucleotide (Microsynth AG) and 3.75 U/reaction Expand High Fidelity Enzyme Mix (part of Expand High Fidelity PCR System; Roche Diagnostics). The PCR reactions were run using the combinations of genomic DNA template, oligonucleotide and PCR protocols as summarized in Table 1.

The PCR reactions were run with one of the following temperature protocols:
Protocol 5.1.1:
   94°C 2min., 35× (94°C 15 sec., 62°C 30 sec., 68°C 10 min.), 68°C 20 min, ∞ 4°C
Protocol 5.1.2:
   94°C 2min., 35× (94°C 15 sec., 63°C 30 sec., 68°C 10 min.), 68°C 20 min, ∞ 4°C
Protocol 5.1.3:
   94°C 2min., 35× (94°C 15 sec., 62°C 30 sec., 68°C 3.5 min.), 68°C 20 min, ∞ 4°C

The PCR products were analyzed by standard agarose gel electrophoresis (Fig. 8) as described in Sambrook et al. (Molecular Cloning: A laboratory manual. 1989, Cold Spring Harbor Laboratory Press, Cold Spring Harbor.). The size and partial sequence proves the expected identity of the DNA fragments and provides evidence that the homologous recombination of the targeting vector with the chromosomal TAAR1 gene locus occurred in a precise manner for the 5' as well as the 3' genomic arm of the targeting vector. The DNA sequencing results summarized in listing 1-9 proves furthermore, that the homologous recombination occurred without undesired side events such as the chromosomal integration of tandem repeats of the vector constructs or parts thereof or a loss of the NLSlacZ coding sequence included in the targeting vector. In addition, the DNA sequence analysis of DNA fragments 1-3 (listing 1, 4 and 5) demonstrates that the NLSlacZ coding sequence was targeted to the *TAAR1* gene locus such that the NLSlacZ open reading frame starts with the endogenous start codon of the *TAAR1* gene and that the structure of the surrounding genomic sequence including putative promotor and other elements involved in transcriptional regulation of the *TAAR1* gene from the TAAR1⁺ allele is preserved in the TAAR1^{NLSlacZ} allele. Consequently, the NLSlacZ transcript shall be expressed from the TAAR1^{NLSlacZ} allele with the same spatio-temporal profile as the TAAR1 transcript from the TAAR1⁺ allele, and NLSlacZ expression in animals carrying the mutant allele shall reflect the endogenous expression profile of TAAR1 in wild type animals. An example for the use of the NLSlacZ expression from the TAAR1^{NLSlacZ} allele as tool for analyzing the TAAR1 expression will be provided below.

For DNA sequence analysis DNA fragments 1-4 were amplified from genomic DNA by PCR and subjected to agarose gel electrophoresis as described above. PCR products of the expected sizes were cut out from the gels with sterile scalpels (Bayha, Tuttlingen, Germany) and extracted from the agarose gel slices using the QIAquick Gel Extraction Kit (QIAGEN AG, Basel, Switzerland) following the instructions of the manufacturer. The extracted PCR products were adjusted to a defined concentration with cold ethanol/sodium acetate DNA precipitation (Sambrook, J., Fritsch, E.F., und Maniatis, T.: Molecular Cloning: A laboratory manual. 1989, Cold Spring Harbor Laboratory Press, Cold Spring Harbor.) and subsequently dissolving the DNA pellets in the required volume of 10 mM Tris/HCl pH 7.5. DNA sequence analysis of the PCR products was carried out at Microsynth AG employing BigDye chemistry and a 3730 capillary DNA analyzer (Applied Biosystems), and the oligonucleotides used for the individual sequence analysis reactions as well the sequence analysis results are summarized in listing 1-9 (see below). The sequence listings are limited to those parts of the sequence analysis results which were judged as reliable based on the chromatograms.

**Table 1: PCR amplification of DNA fragments 1-4 (see Fig. 7) from genomic DNA derived from tail biopsies of TAAR1+/+ and TAAR1^{NLSlacZ/NLSlacZ} mice. The obtained pattern of PCR products supported by the results of partial DNA sequence analysis of the DNA fragments confirms the correct targeting of the TAAR1 gene in the TAAR1^{NLSlacZ} allete**

| PCR reaction | Oligonucleotide combination | Template TAAR1^{+/+} genomic DNA | Template TAAR1^{NLSlacZ} genomic DNA |
|---|---|---|---|
| PCR protocol 5.1.1 | mTAR31c (5'-gaaggtggaattctaacctgac-3') | • PCR product: DNA fragment 1 (2.72 kb, see Fig. 7 & 8) | **•** PCR product: DNA fragment 3 (5.05 kb; see Fig. 7 & 8) |
| | mTAR32nc (5'- ctcatgtgaatcagtaccacag-3') | • DNA sequence analysis (listing 1) fits expected sequence | • DNA sequence analysis (listing 2) fits expected sequence |
| PCR protocol 5.1.2 | mTAR24c (5'-gtgggctaagatctaggaacg-3') | • No PCRproduct (as expected, see Fig. 7 & 8) | • PCR product: DNA fragment 2 (7.33 kb; see Fig. 7 &8) |
| | lacZ10 (5'-ggaacaggtattcgctggtcac-3') | | • Sequence analysis (listing 2-4) fits expected sequence |
| PCR protocol 5.1.3 | PGK1 (5'- gtgggctctatggcttctgag-3') | • No PCR product (as expected, see Fig. 7 & 8) | • PCR product: DNA fragment 4 (2.86 kb; see Fig. 7 & 8) |
| | mTAR25nc (5'- aggtccaactctgtgtgatgg-3') | | • Sequence analysis (listing 7-9) fits expected sequence |

| Listing 1 (SEQ. ID NO:1): Result of DNA sequence analysis of DNA fragment 1 (Fig. 7/Table 1) with oligonucleotide mTAR31c (Table1) |
|---|
| 1 GAGGGAAAGC CCAGCCTGTG TCTAGTTCTC TGCAGTGATG CATCTTTGCC 51 ACGCTATCAC AAACATTTCC CACAGAAACA GCGACTGGTC AAGAGAAGTC 101 CAAGCTTCCC TGTACAGCTT AATGTCACTC ATAATCCTGG CCACTCTGGT 151 TGGCAACTTA ATAGTAATAA TTTCCATATC CCATTTCAAG CAACTTCATA 201 CACCCACCAA CTGGCTCCTT CACTCCATGG CCATTGTCGA CTTTCTGCTG 251 GGCTGTCTGA TAATGCCCTG CAGCATGGTG AGAACTGTTG AGCGCTGTTG 301 GTATTTTGGG GAAATCCTCT GTAAAGTTCA CACCAGCACC GATATCATGC 351 TGAGCTCCGC CTCCATTTTC CACTTAGCTT TCATTTCCAT TGACCGCTAC 401 TGTGCTGTGT GTGACCCTTT GAGATACAAA GCCAAGATCA ATATCTCCAC 451 TATTCTTGTG ATGATCCTCG TTAGTTGGAG CCTTCCTGCT GTTTATGCAT 501 TTGGGATGAT CTTCCTGGAA CTGAACTTAA AAGGAGTGGA AGAGCT |

| Listing 2 (SEQ. ID NO:2): Result of DNA sequence analysis of DNA fragment 2 (Fig. 7/Table 1) with oligonucleotide mTAR24c (Table1) |
|---|
| 1 GACATTTTAT TTACAGGCAC AGAGTCTCCT GGACAGGCTG GTGAAGGTGA 51 ACTCTGATTC AAAGACCGTG TGAGGGGTAT CCACCAGAGA AGACCGCTGG 101 GACCAGGGCT TAAGTCTTTA TTAGCAGGTC GGTGTCTACA CTGGGTGTTT 151 GGGATCCCAG TGTAGTCCCG AGCCTTTCTT TGAGCCTTTA AGCACAAAAA 201 AAAACCAAAA AACTAGTTTC AGGGTTGACA TACTTCAGTT ACCAAGAACA 251 GTTAGCCAGA AGTGGAACTA TAAAAGCCAA ATAGTAAGGT TAATACATTT 301 GGAAACTTTC CCAGGCCTTT GATGGATTAG GTCTGTGTTT TAGTTTTGGC 351 AGCCAGTGCT ATGTGCTGAG TTTGGCAGCC TGAGTGATGT GTCTATCATG 401 GAGTCCATCA TGCTAAGGTG TCAGGGTCTA CTAAAGTCTG TGGGGCTGTT 451 ACAAGGCCAT ATTCATGTAA AACCTACACA AACGTGCCAT TCTATAAAAG 501 ACGGTTACAT AACAGGTATA TCATGATTTA CTTTCGGGTT CTCTAGATAT 551 CTGGTGTCAG TCATTTTGTG CTGACTCATG GTGGTCCTGA AAAAGTATAA 601 CGTAAAAGCA AAATTCTAGG CCTTTAGGCC CAGGCTTGAG AATGTGACCT 651 CTGTGACTCA ATGCTCCAAG GTATGCTAAT CATAAAACAG ATAGCGACAT 701 TCCTCCACCC ACCCTCTTCC TGGGTTCAAG GAGTGTTCAT TT |

| Listing 3 (SEQ. ID NO:3): Result of DNA sequence analysis of DNA fragment 2 (Fig. 7/Table 1) with oligonucleotide mTAR39c (5'- cactcttacatccagccttagc- 3') |
|---|
| 1 TGTTCTTCTC TAAGAGCCTG TCACTCACCG GCATTCGGCA ACCTTCATCG 51 TTGTTGGTTT GTAAACAAGT GTGGGGATGC TCTTTGACAT TTTCATTTCT 101 ATAATGTTTA GTACGTTCAC TGTCCATCAG ATCAGTATGA TATTTAGTCA 151 CGATTTCATT ATTCGCTTTT GTACTTTCCT GGAGAAATTA AAACACCACA 201 TACCATTTCT TTAGACCATT CACATTTATC TTCTTAGTTA GTGTGCTTTA 251 TCTGTTTTTG GCTTTAGATT TTCATTTCTC TGTCTCCACA GTCCTTTCTT 301 ATTTGACTTG GCTTCTGTCT GCTGTCATTC CACATTGCTA ATGGTGTTTT 351 AATGTTTCTT GGCAACACAT CTCAGGAGCA CGGGATTTGG AGCATCTCTA 401 AGATTCTGCT ATCGGGGTCT CTTCAGTAGA TTCTCTTTCA CTGACAAAGC |
| 451 TTACTGTCAG TGCTTGAGAG CTCACTCCAC ACTTGGTTGT TACACATGCA 501 GCTCTCCCGT AATTGCATTT GTATTTTAAG GCAGAACTTG TTGCAATTAT 551 TGAAACACAA TTCTCTTTGA TTTGCATAGA ATTAATTATT TGGTTTTGTT 601 TAGAACCCAG TTCTCAACAT CTTAATTTTC CTGTCTTCTT CAAGCAATAA 651 AGGACACATA GGTGTTGTAT CTTGTGATTC CTTGATTCTG AGAGACATCT 701 TTCACACTTA CACAAAGACA TTTCTTTGTT CTTTGTTAAA TATTCTTACA 751 TTTAACATTC TCTTGTTTTA AACAAGTTCC TTGTAGCTCT CTATGTTGTT 801 TCTTATTAAA TGAAAGGCTC AGCATTCCTG AGACATTTAA GGCTATGTGA 851 CTCTGGGAAA ACACATTAAT TAA |

| Listing 4 (SEQ. ID NO:4): Result of DNA sequence analysis of DNA fragment 2 (Fig. 7/Table 1) with oligonucleotide mTAR31c (Table 1) |
|---|
| 1 GGGAAAGCCC AGCCTGTGTC TAGTTCTCTG CAGTGATGCA TCCAAAGAAG 51 AAGAGAAAGG TTTCGGAGGG GGGGAGCTTG ATGATCTGTG ACATGGCGGA 101 TCCCGTCGTT TTACAACGTC GTGACTGGGA AAACCCTGGC GTTACCCAAC 151 TTAATCGCCT TGCAGCACAT CCCCCTTTCG CCAGCTGGCG TAATAGCGAA 201 GAGGCCCGCA CCGATCGCCC TTCCCAACAG TTGCGCAGCC TGAATGGCGA 251 ATGGCGCTTT GCCTGGTTTC CGGCACCAGA AGCGGTGCCG GAAAGCTGGC 301 TGGAGTGCGA TCTTCCTGAG GCCGATACTG TCGTCGTCCC CTCAAACTGG 351 CAGATGCACG GTTACGATGC GCCCATCTAC ACCAACGTGA CCTATCCCAT 401 TACGGTCAAT CCGCCGTTTG TTCCCACGGA GAATCCGACG GGTTGTTACT 451 CGCTCACATT TAATGTTGAT GAAAGCTGGC TACAGGAAGG CCAGACGCGA 501 ATTATTTTTG ATGGCGTTAA CTCGGCGTTT CATCTGTGGT GCAACGG |

| Listing 5 (SEQ. ID NO:5): Result of DNA sequence analysis of DNA fragment 3 (Fig. 7/Table 1) with oligonucleotide mTAR31c (Table 1) |
|---|
| 1 GAGGGAAAGC CCAGCCTGTG TCTAGTTCTC TGCAGTGATG CATCCAAAGA 51 AGAAGAGAAA GGTTTCGGAG GGGGGGAGCT TGATGATCTG TGACATGGCG 101 GATCCCGTCG TTTTACAACG TCGTGACTGG GAAAACCCTG GCGTTACCCA 151 ACTTAATCGC CTTGCAGCAC ATCCCCCTTT CGCCAGCTGG CGTAATAGCG 201 AAGAGGCCCG CACCGATCGC CCTTCCCAAC AGTTGCGCAG CCTGAATGGC 251 GAATGGCGCT TTGCCTGGTT TCCGGCACC |

| Listing 6 (SEQ. ID NO:6): Result of DNA sequence analysis of DNA fragment 3 (Fig. 7/Table 1). The listed sequence represents a contig assembled from sequence analyses of DNA fragment 3 with oligonucleotides mTAR32nc (Table 1) and IL4rev (5'- ggcgatagaaggcgatgcgc- 3') |
|---|
| 1 TCATCTCCGG GCCTTTCGAC CTGCAGCCAA TATGGGATCG GCCATTGAAC 51 AAGATGGATT GCACGCAGGT TCTCCGGCCG CTTGGGTGGA GAGGCTATTC 101 GGCTATGACT GGGCACAACA GACAATCGGC TGCTCTGATG CCGCCGTGTT 151 CCGGCTGTCA GCGCAGGGGC GCCCGGTTCT TTTTGTCAAG ACCGACCTGT 201 CCGGTGCCCT GAATGAACTG CAGGACGAGG CAGCGCGGCT ATCGTGGCTG 251 GCCACGACGG GCGTTCCTTG CGCAGCTGTG CTCGACGTTG TCACTGAAGC 301 GGGAAGGGAC TGGCTGCTAT TGGGCGAAGT GCCGGGGCAG GATCTCCTGT 351 CATCTCACCT TGCTCCTGCC GAGAAAGTAT CCATCATGGC TGATGCAATG 401 CGGCGGCTGC ATACGCTTGA TCCGGCTACC TGCCCATTCG ACCACCAAGC 451 GAAACATCGC ATCGAGCGAG CACGTACTCG GATGGAAGCC GGTCTTGTCG 501 ATCAGGATGA TCTGGACGAA GAGCATCAGG GGCTCGCGCC AGCCGAACTG 551 TTCGCCAGGC TCAAGGCGCG CATGCCCGAC GGCGATGATC TCGTCGTGAC |
| 601 CCATGGCGAT GCCTGCTTGC CGAATATCAT GGTGGAAAAT GGCCGCTTTT 651 CTGGATTCAT CGACTGTGGC CGGCTGGGTG TGGCGGACCG CTATCAGGAC 701 ATAGCGTTGG CTACCCGTGA TATTGCTGAA GAGCTTGGCG GCGAATGGGC 751 TGACCGCTTC CTCGTGCTTT ACGGTATCGC CGCTCCCGAT TCGCAGCGCA 801 TCGCCTTCTA TCGCCTTCTT GACGAGTTCT TCTGAGGGGA TCAATTCTCT 851 AGAGCTCGCT GATCAGCCTC GACTGTGCCT TCTAGTTGCC AGCCATCTGT 901 TGTTTGCCCC TCCCCCGTGC CTTCCTTGAC CCTGGAAGGT GCCACTCCCA 951 CTGTCCTTTC CTAATAAAAT GAGGAAATTG CATCGCATTG TCTGAGTAGG 1001 TGTCATTCTA TTCTGGGGGG TGGGGTGGGG CAGGACAGCA AGGGGGAGGA 1051 TTGGGAAGAC AATAGCAGGC ATGCTGGGGA TGCGGTGGGC TCTATGGCTT 1101 CTGAGGCGGA AAGAACCAGC TGGGGCTCGA TCCTCTAGAG TCGATCGACC 1151 TCGACCCCCC CTATGCATTT TTGGAGAATC ATCTATACTT TACAGTGAGT 1201 TTCTGTGGCT TTATTTATTT CAAGATCTTC CTTACAGATT ATT |

| Listing 7 (SEQ. ID NO:7): Result of DNA sequence analysis of DNA fragment 4 (Fig. 7/Table 1) with oligonucleotide PGK1 (Table 1) |
|---|
| 1 CGATCGACCT CGACCCCCCC TATGCATTTT TGGAGAATCA TCTATACTTT 51 ACAGTGAGTT TCTGTGGCTT TATTTATTTC AAGATCTTCC TTACAGATTA 101 TTTCCAGATG TTGTATAAAT CTTTTAAAAA CTGTGGTACT GATTCACATG 151 AGAAAAAAGG CTCTAGAAAT GAAGAGCCCA AGATCCAGAA ATAACTGTCT 201 GGGTTTATGA GCAGTTGTGT AGCTGTGTGA TCAGAGGTTG ATTATGTACA 251 GTCTAAGGGG AGTAGCTGAG TGAAAAGGAA AAAGACTAGT TTTTAATGTA 301 ACCATTATTG CCACTATTAT ATATGTTATA TAAACAAGGT TATGTATAAA 351 CAATAAAGTT TATATTATAT TTTCACTTGT TTTGTTTCTT GTTTTATAAT 401 ATATAGGCAT ATTAAACTTT ACATTGTTTT AACTTTTTTT AAATAATTGC 451 TATTATTTTT TTGAGACAGG GTCTTAGTAT ATAGCTTGAC TAGCCCAGAA 501 GTCTCTGTGA AGACTAGGAT GGTCTCAGAC TCAGAGAGAT TTGCCTGCCC 551 CTGCTGCCCA AGTGCTGAGA TTTAAGTATT TAGTCATTAA GGTTTGTATA 601 TACATTATGT CTAGCCATTG AGGTTTTTAT ATTCACCACA TCCAGATGTT 651 AAGGTTTCTA GGCACACATC CATTTCTTAC TCTATTAGTC AGAATTTCTA 701 GAACAATACT GATATTAAGG ATGGATCTTA TTACTTGG |

| Listing 8 (SEQ. ID NO:8): Result of DNA sequence analysis of DNA fragment 4 (Fig. 3/Table 1) with oligonucleotide mTAR1D11 (5'- atagggaacttttgggatagc- 3') |
|---|
| 1CCTACCTCTG GCCTCTGGCC TTTCCCTTTA ACTCCACAGA TAAGTGCATA 51AGCTCCTTTA AAAATTTCAG AAGATTTTTT TTCCAGAATC TTTATAAATG 101TAAGCAGGCA GTGATCCTTC ACATTATCAT TAGGAGGGAC TAGCCAAAGG 151GTGTAGAGTT CCAGTTTGCA CAAGGGCTAA GGTCATGGTC AGACAGCTCC 201CTCTATGTCC ATTTCCAATG TGAGTTACGA AACTTCTTAC CAAGTTCATG 251AGTTCCAGAC AGCCTTCCAA GAAAAGTTTT TAGTTACATC AATGGAAAAG 301ATAGAATCTG GTTGTTGCAA GAAAAACAGT TACAACAACA TCAATACTTA 351TTTCTAAGTC AATTTGTGGC AAAAATAATT TCATTTCGCA GGTTCTACTG 401AAAATATTCA TATAAATGTT CATTTCATAT CAAGTAGTAA TGTGTTGGTC 451AGGACTTTTT TGTGGAAATG GATTATTAAC AACGGTGCTT TCTTAAGAGG 501TTAGTGAGCT AGGAGGTTCC ATCATGATTT TAGACTATAT CTGGAAGTCA 551ACAATCCAGT CTTGGCAAAC TCACACATAT TTGTCAGAAA GTAGGACAAA 601GTGGGTAATC TCCAATCCAC TGAGAATTTT GGTAAGTCAC AGTTTCTGCT 651TAGCTGAACC AGAGTCAAGA AGAGCTTGAA TATTAAGGGT AGAAAGATGC 701ATGAAGAATG GTGGATTTGG TTTTTCTAAA ACAGTTTGCT TTGAATATTT 751 ATTTTAGTTA TTTTTTGGGG GGAGGAAAGT TTATTTAGCT TACACT |

| Listing 9 (SEQ. ID NO:9): Result of DNA sequence analysis of DNA fragment 4 (Fig. 7/Table 1) with oligonucleotide mTAR25nc (Table 1) |
|---|
| 1 GGCATTCCCC TGTACTGGGG CATATAAAGT TTGCAATACC AAAGGTCCTC 51 TCCTCCCAGT GATGGCCGAT TAGGCCATCT TCTGCTACAT ATGCAGCTAG 101 AGATATGAAG TCATTAGATA TGCACTCACT GATAAGTGGA TATTAGCCCA 151 GAAACATAGA ACACCCAAGA TACAATTTGC AAAACACAAG AAAATCAAGA 201 AGAAGGAAGA CCAATGGATG GATACTTCAT TCCTCCTTAG AATAGGGAAC 251 AAAATACCCA TGAAAGGAGT TACAGAGACA AAGTTTGGAG CTAAGACAAA 301 AGGATGGACT ATCCAGAGAC TAGCCCTCCC AGGGATCCAT CCCATAATCA 351 GCCACCAAAC CTAGACACTA TTGCATATGC CAGAAAGATT TTGCTGAAGG 401 GACCCTGTTA TAGCTGTCTC GTATGAGGCT ATGCCAGTGC CTGGCAAACA 451 CAGAAGTGGA TGCTCACAGT CATCTATAAG ATGGAACTCA GGGCCCCCAA 501 TGGAGGAGCA AGAGAAAGCA CCCAAGAAGA TGAAGGGGTC TGCAACCCTA 551 TAGGTGGAAC AACAATATGA ACTAACCAGT ACCCCCAGAG CTCATATCTC 601 TAGCTGCATA TATTAGTTAT TTTAAAGTTG TGTGTGCATG CATGTGTGTG 651 CACGTGGGAA CAGAAGTGTG GGTGCCAGTA GAGGAATTTC AATCCCTTGC 701 AGCTAGAATT ACAGGGAGAC TTGAATTGCC CACAAGATTG CTGGGAACTG 751 ACTCTGGGTC CTCAGTAAGA GCTGCATTT |

In order to confirm the successful gene replacement on the transcript level cDNA derived from whole brains of juvenile TAAR1^{+/+} or TAAR1^{NLSlacZ/NLSlacZ} mice were prepared as described below.

RNA was prepared from tissue samples essentially according to Chomczynski and Sacchi (Single-step method of RNA isolation by acid guanidinium thiocyanate-phenolchloroform extraction, Anal. Biochem. 162 (1987) 156-159.). In brief, mouse pups of day 1l after birth were sacrificed, and total brains were removed and homogenized in the 10× volume of Trizol Reagent (Invitrogen, Paisley, UK) in a glass douncer (Inotech AG, Dottikon, Switzerland). Total RNA was isolated according to the instructions of the manufacturer. Raw RNA was dissolved in 10 mM Tris/HCl pH 7.0 and treated with 25 U/µl DNAse I (Roche Diagnostics, Rotkreuz, Switzerland) in 10 mM Tris/HCl pH 7.0 with 10 mM MgCl₂ for 1 hr. The DNAse I was removed by phenol/chloroform extraction according to (Sambrook, J., Fritsch, E.F., und Maniatis, T.: Molecular Cloning: A laboratory manual. 1989, Cold Spring Harbor Laboratory Press, Cold Spring Harbor.). RNA was precipitated, dissolved to a final concentration of 1 mg/ml in 10 mM Tris/HCl pH 7.0 and used for first strand synthesis.

For cDNA synthesis Superscript II RNAse H⁻ Reverse Transcriptase (Invitrogen, Paisley, UK) was used according to the instructions of the manufacturer. Briefly, 4 µg total RNA were mixed with 1 µg oligo (dT)₁₂₋₁₈ (Invitrogen, Paisley, UK) in H₂O in a total volume of 23 µl, incubated at 70°C for 10 min. and chilled on ice. The following components were added on ice (final concentrations): 50 mM Tris-HCl (pH8.3), 75 mM KC1, 3 mM MgCl₂, 10 mM DTT, 0.5 mM of each dNTP (Amersham, Otelfingen, Switzerland), 1 U/µl RNAse Out RNAse inhibitor (Invitrogen) and 10 U/µl reverse transcriptase. The reaction was incubated for 1 hr at 42°C, stopped by incubation at 70°C for 10 min. and diluted to a total volume of 100 µl with 10 mM Tris/HCl pH 7.0.

PCR amplification of transcripts was carried out essentially as described in 4, but with the following modifications: PCR reactions were carried out in a total volume of 50 µl per reaction composed as follows (final concentrations/amounts): 1 µl of cDNA preparation (equivalent to a total amount of 40 ng whole brain total RNA), 20 mM Tris-HCl (pH8.4), 50 mM KCl, 1.5 mM MgCl₂, 200 nM of each oligonucleotide (Microsynth AG, Balgach, Switzerland), 200 mM of each dNTP (Amersham), 5 U/reaction recombinant Taq DNA polymerase (Invitrogen). For the detection of individual mRNA transcripts the following oligonucleotides and temperature profiles were used:
Glyceraldehyde-3-phosphate dehydrogenase (GAPDH):
   Oligonucleotides:
      GAPDH U: 5'-accacagtccatgccatcac-3' (SEQ. ID NO: 24);
      GAPDH D: 5'-tccaccaccctgttgctgta-3' (SEQ. ID NO: 25)
   Temperature profile:
      94°C 2min., 22× (94°C 15 sec., 64°C 30 sec., 72°C 1 min.), 72°C 5 min, ∞ 4°C
TAAR1:
   Oligonucleotides:
      mTAAR1 U1: 5'-atgcatctttgccacgctatc-3'(SEQ. ID NO: 26);
      mTAAR1 D2: 5'-caaggctcttctgaaccagg-3' (SEQ. ID NO: 27)
   Temperature profile: 94°C 2min., 40× (94°C 15 sec., 53°C 30 sec., 72°C 1 min.), 72°C 5 min, ∞ 4°C
NLSlacZ:
   Oligonucleotides:
      VN12taulacZ U1: 5'- ggtggcgctggatggtaa-3' (SEQ. ID NO: 28);
      VN12taulacZ D1: 5'- cgccatttgaccactacc-3' (SEQ. ID NO: 29)
   Temperature profile: 94°C 2min., 40× (94°C 15 sec., 60°C 30 sec., 72°C 1 min.), 72°C 5 min, ∞ 4°C

As expected, the GAPDH transcript was detected in both cDNA preparations from TAAR1^{+/+} and TAAR1^{NLSlacZ/NLSlacZ} mouse brain indicating that the cDNApreparations per se were successful. While the PCR analysis detected TAAR1 transcript only in TAAR1^{+/+}, but not in TAAR1^{NLSlacZ/NLSlacZ} mouse brain cDNA, the NLSlacZ transcript was detected only in TAAR1^{NLSlacZ/NLSlacZ}, but not in TAAR1^{+/+} mouse brain cDNA.

These data reveal that there is no TAAR1 expression in TAAR1^{NLSlacZ/NLSlacZ} mouse brain which is in agreement with the deletion of the TAAR1 coding sequence in the TAAR1^{NLSacZ/NLSlacZ} mouse line. The presence of NLSlacZ mRNA transcript in TAAR1^{NLSlacZ/NLSlacZ} mouse brain provides evidence for the expression of NLSlacZ in TAAR1^{NLSlacZ/NLSlacZ} mutants from the endogenous TAAR1 gene locus. The absence of NLSlacZ transcript from TAAR1^{+/+} mouse brain as apparent from Fig. 9 C is in line with the fact that NLSlacZ does not naturally occur in mammalian species and supports the specificity of the PCR conditions.

**Table 2: Results of the analysis of cDNAs derived from TAAR1^{+/+} and TAAR1^{NLSlacZ/NLSlacZ} mouse brain for the presence of GAPDH, TAAR1 and NLSlacZ mRNA transcripts.**

| | | |
|---|---|---|
| PCR specific | cDNA used as PCR template | |
| for transcript | TAAR1^{+/+} brain cDNA | TAAR1^{NLSlacZ/NLSlacZ} brain cDNA |
| GAPDH | 452 bp fragment | 452 bp fragment |
| TAAR1 | 936 bp fragment | no PCR product |
| NLSlacZ | no PCR product | 631 bp fragment |

### 4.2) Analysis of the genetic background of the TAAR1 - NLSlacZ knock-in mouse line

The genetic background of genetically modified mouse lines has a profound impact on their phenotype, and the variability of the genetic background between individual animals of a mouse line caused e.g. by a so-called mixed genetic background can complicate or even make impossible the meaningful and consistent phenotypical characterization of a mutant mouse line or its use e.g. in behavioral pharmacology (Gerlai, R., Gene-targeting studies of mammalian behavior: is it the mutation or the background genotype? Trends Neurosci. 19 (1996) 177-181.; Bucan and Abel, The mouse: genetics meets behaviour. Nat. Rev. Genet. 3 (2002)114-123; Banburry Conference on Genetic Background in Mice (1997): Mutant Mice and Neuroscience: Recommendations Concerning Genetic Background. Neuron 19, 755-759). For historical and practical reasons, targeted mouse mutants are most frequently generated using ES cells derived from one of the various SV129 inbred mouse lines (Hogan, B., Beddington, R., Costantini, F., und Lacy, E.: Manipulating the mouse embryo. 1994, Second Edition, Cold Spring Harbor Press, Cold Spring Harbor.; Threadgill, D.W., Yee, D., Matin, A., Nadeau, J.H., Magnuson ,T., Genealogy of the 129 inbred strains: 129/SvJ is a contaminated inbred strain. Mamm Genome. 8 (1997) 390-393). Because of the unfavorable properties of the SV129 mouse lines related to neuroanatomy, breeding performance and behavior mice generated using SV129 ES cells need to be transferred to a homogenous and more favorable genetic background by backcrossing with mice of the desired genetic background for at least 10 generations requiring several years of work (Silver, L.M.: Mouse Genetics. 1995, Oxford University Press, New York).

The use of ES cells derived from C57BL/6 mice (Kontgen, F., Suss, G., Stewart, C., Steinmetz, M., Bluethmann H., Targeted disruption of the MHC class II Aa gene in C57BL/6 mice. Int Immunol. 5 (1993) 957-964) in combination with an appropriate breeding scheme allowed us to generate a mutant mouse line carrying the TAAR1^{NLSlacZ} allele on a pure C57BL/6 genetic background. The homogeneity of the genetic background was experimentally confirmed by means of microsatellite analysis on 5 heterozygous mutants of the F1 generation as well as for the ES cell line used for the generation of germline chimeras.

For this analysis genomic DNA was isolated from tail biopsies of 5 mice of the F1 generation carrying the TAAR1^{NLSlacZ} allele, as well as from a sample of ES cells used for the generation of the germline chimeras, with the MagNAPure LC system for nucleic acid purification (Roche Diagnostics, Basel, Switzerland). Genomic DNA of congenic C57BL/6, DBA and SV129 mice were used as standard and were analyzed in parallel by PCR; the genomic DNA of the congenic inbred mouse strains C57BL/6, DBA and SV129 were purchased from Jackson Laboratory (Bar harbor, Maine, USA).

PCR reactions were performed on a GenAmp 9700 thermocycler (Applied Biosystems) in a total volume of 20 µl per reaction composed as follows (final concentrations/amounts) :

10-20 ng genomic DNA, 67 mM Tris-HCl pH 8.8, 16.6 mM (NH₄)₂SO₄, 0.1 mg/ml BSA, 2 mM MgCl₂, 200 µM of each dNTP (Amersham), 200 nM of each oligonucleotide (Microsynth AG) and 1U/reaction Taq DNApolymerase (Invitrogen). All microsatellite PCR reactions were run with the following temperature profile:

94°C 2min., 35× (94°C 15 sec., 55°C 45 sec., 72°C 1 min.), 72°C 5 min, ∞ 4°C. PCR products were analyzed using an Elchrom electrophoresis unit (Elchrom Scientific AG, Cham, Switzerland).

For confirming the homogeneity of the genetic background of the mutant mouse line carrying the TAAR1^{NLSlacZ} allele a density of about 2 markers pro chromosome were used, and the following microsatelites were included into the analysis: D1MIT217, D1MIT291, D2MIT312, D2MIT285, D3MIT22, D3MIT45, D4MIT149, D4MIT166, D5MIT259, D5MIT95, D6MIT86, D6MIT188, D7MIT76, D7MIT246, D8MIT155, D8MIT248, D9MIT191, D9MIT182, D10MIT35, D10MIT83, D11MIT149, D11MIT99, D12MIT136, D12MIT99, D13MIT16, D13MIT35, D14MIT203, D14MIT165, D15MIT193, D16MIT131, D16MIT4, D17MIT93, D17MIT155, D18MIT19, D18MIT152, D19MIT71, DxMIT64 (oligonucleotide sequences were chosen according to Jaxon Lab Mouse Informatics Database; Eppig, J.T. et al. The Mouse Genome Database (MGD): from genes to mice - a community resource for mouse biology. Nucleic Acids Res. 33, D471-D475 2005).

The microsatellite analysis revealed that the mutant mouse line carrying the TAAR1^{NLSlacZ} allele matches with wild type C57BL/6 mice for all microsatellites tested (see Fig. 10 for example), confirming that the mutant mouse carrying the TAAR1^{NLSlacZ} allele possesses a pure C57BL/6 genetic background and harbors no potential contaminations from either DBA or SV129.

### 4.3) Proof of concept: Use of TAAR1 - NLSlacZ gene replacement as tool for analyzing the tissue distribution of TAAR1 expression

The TAAR1^{NLSlacZ} mutant mouse line was generated using a gene replacement strategy as described in chapter 1). As a consequence, the histological marker NLSlacZ has been targeted to the *TAAR1* gene locus such that its expression reflects the spatiotemporal tissue distribution of TAAR1 expression in wild type animals. To this end, the TAAR1^{NLSlacZ} mutant mouse line serves as a powerful tool which allows for detailed TAAR1 expression studies without the need to generate and validate TAAR1-specific probes such as specific antibodies or radioligands.

The expression of a synthetic coding sequence from a chromosomal locus can be potentially compromised e.g. by gene silencing events or by insufficient expression levels, both of which are difficult to predict without experimental data derived from the actual mutant of interest. In order to proof the functionality of the NLSlacZ coding sequence targeted to the TAAR1 gene locus in the TAAR1^{NLSlacZ} mutant mouse as histological marker a lacZ staining was carried out on tissue sections of adult TAAR1^{NLSlacZ/NLSlacZ} and TAAR1^{+/+} mouse brains.

Adult TAAR1^{NLSlacZ/NLSlacZ} and TAAR1^{+/+} mice were transcardially perfused under terminal isoflurane anesthesia essentially as described in Romeis (Mikroskopische Technik. 1989, 17., neubearbeitete Auflage, Urban and Schwarzenberg; Minchen, Wien, Baltimore). The animals were perfused consecutively with 10 ml phosphate buffered saline (PBS; 137 mM NaCl, 2.7 mM KCl, 90 mM Na₂HPO₄, 1,5 mM KH₂PO₄, pH 7.4) and 15 ml fixative (2% w/v paraformaldehyde and 0.2% w/v glutaraldehyde in PBS). The brains were removed from the skull, post-fixed for 4 hours in fixative at 4°C and immersed into 0.5 M sucrose in PBS over night at 4°C. Brains were embedded in OCT compound (Medite Medizintechnik, Nunningen, Switzerland) in Peel-A-Way tissue embedding molds (Polysciences Inc., Warrington, USA) and frozen on liquid nitrogen. Brains were cut in parasagittal orientation on a cryomicrotome (Leica Microsystems AG, Glattbrugg, Switzerland) at 50 µm and thaw mounted on gelatin coated glass slides (Fisher Scientific, Wohlen, Switzerland). Tissue sections were air dried at room temperature for 2 h, washed 5 times for 10 min each in PBS at room temperature (RT) and incubated for 16-24 h in lacZ staining solution (1 mg/ml 5-bromo-4-chloro-3-indolyl-beta-D-galactopyranoside, 5 mM K₃Fe(CN)₆, 5 mM K₄Fe(CN)₆, 2 mM MgCl₂ in PBS) in a light tight container at 37°C on a horizontal shaker. The staining process was stopped by washing the tissue sections 5 times for 10 min each in PBS at RT. Tissue sections were dehydrated through an ascending ethanol series, equilibrated to xylene and coverslipped with DePex (Serva GmbH, Heidelberg, Germany). Tissue sections were analyzed on an Axioplan I microscope (Carl Zeiss AG, Feldbach, Switzerland) equipped with an Axiocam CCD camera system (Carl Zeiss AG).

The lacZ staining of the tissue sections revealed a strong and specific staining in TAAR1^{NLSlacZ/NLSlacZ} mouse brain sections, which was absent from a tissue section of an equivalent region of a TAAR1^{+/+} mouse brain. This result confirms, that NLSlacZ expression from the *TAAR1* gene locus in TAAR1^{NLSlacZ} mutant mice functions as histological marker for analyzing TAAR1 expression in mouse (Figure 11).

In order to exclude potential deficits in the spontaneous behavior or sensory capabilities and the physiological conditions of the TAAR1^{NLSlacZ} mutant mouse line, which could arise through the mere presence of the targeted mutation of the *TAAR1* gene. The baseline conditions were analyzed in adult animals 3 months of age of all three genotypes and both genders.

To this end, the physical state of animals was examined regarding gain of body weight in the first three months of postnatal age, regarding rectal temperature, and nest building behavior. The neurological state of the animals was analyzed regarding the potential occurrence of catalepsy, ataxia, tremor, lacrimation and salivation and the degree of arousal in response to transfer of the animals to a novel environment. The dexterity and coordination of the animals was examined by analyzing grip strength and spontaneous horizontal locomotor activity as well as in the so-called rotarod and horizontal wire tests (e.g. Crawley, J.N. (2000) What's Wrong With My Mouse? 1. Edition, Wiley & Sons, ISBN 0471316393; Irwin, S. Comprehensive observational assessment: Ia. A systematic, quantitative procedure for assessing the behavioral and physiologic state of the mouse. Psychopharmacologia 13, 222-257, 1968).

None of the tests and examinations revealed a significant difference in comparison of the genotypes, demonstrating that there are no deficits in spontaneous behavior, sensory capabilities or physiological state which could impact the characterization of the mutant mouse line in behavioral models directed towards dissecting the potential role of TAAR1 in disease models or towards the characterization of pharmacological compounds.

### Example 2:

### METHODS

### Behavioral phenotyping

Animals were maintained under conditions of constant temperature (22±2°C) and humidity (55-65%), and all mice were singly housed for the duration of study. Food and water were available *ad libitum.* All experiments were conducted during the light phase of the light/dark cycle (lights on: 6 a.m.-6p.m.). All animal procedures were conducted in strict adherence to the Swiss federal regulations on animal protection and to the rules of the Association for Assessment and Accreditation of Laboratory Animal Care International (AAALAC), and with the explicit approval of the local veterinary authority.

*Behavioral assessment.* Mice were assessed for standard physiological parameters (body temperature, evolution of body weight), and in several neurological and behavioral tests, including grip strength (g), horizontal wire test, rotarod test, and locomotor activity.

*Body temperature* was measured to the nearest 0.1°C by a HANNA instruments thermometer (Ronchi di Villafranca, Italy) by inserting a lubricated thermistor probe (2 mm diameter) 20 mm into the rectum; the mouse was hand held at the base of the tail during this determination and the thermistor probe was left in place until steady readings were obtained (~ 15 s).

*Body weight* (g) was checked at various time points in mice aged from 12 to 24 weeks.

*Horizontal wire test:* mice were held by the tail and required to grip and hang from a 1.5 mm in diameter bar fixed in a horizontal position at a height of 30 cm above the surface for a maximum period of 1 min. The latency for the mice to fall was measured, and a cut-off of either 60 sec or the highest fall latency score from three attempts was used.

*Rotarod test:* The apparatus consisted of a fixed speed rotarod (Ugo Basile, Biological Research Apparatus, Varese, Italy) rotating at either 16 or 32 rpm. Bar is 10 cm wide, 3 cm in diameter, and 25 cm above the bench. Motor incoordination on the rotating rod translates into animals falling off the bar. On the test day, subjects were placed on the rotarod and their latency to fall measured. All mice were tested at both 16 and 32 rpm, and in both tests a cut-off of either 120 s or the highest fall latency score from three attempts was used.

*Locomotor activity.* A computerized Digiscan 16 Animal Activity Monitoring System (Omnitech Electronics, Colombus, OH) was used to quantitate spontaneous locomotor activity. Data were obtained simultaneously from eight Digiscan activity chambers placed in a soundproof room with a 12 hr light/dark cycle. All tests were performed during the light phase (6 a.m. to 6 p.m.). Each activity monitor consisted of a Plexiglas box (20 x 20 x 30.5 cm) with sawdust bedding on the floor surrounded by invisible horizontal and vertical infrared sensor beams. The cages were connected to a Digiscan Analyzer linked to a PC that constantly collected the beam status information. With this system, different behavioral parameters could be measured, such as horizontal and vertical activity, total distance travelled (in cm), and stereotypies. The mice were tested via a pseudo-Latin squares design twice weekly with at least a 10-day interval between two consecutive test sessions. Vehicle (saline 0.9%) or d-amphetamine (0.4, 1, 2.5, 5 mg/kg, i.p.) was administered to wild-type (n=16) and TAAR-1 KO (n =12) mice just prior to testing. Locomotor activity was recorded for 90 min starting immediately after the mice were placed in the cages.

*Statistics.* Behavioral observations were recorded as mean values SE and analyzed with an unpaired t test. Locomotor activity data (total distance) were analyzed with a two-factor (Genotype and Dose) ANOVA with repeated measures. Comparisons of dose effects in each genotype were undertaken with a repeated measures ANOVA, followed in significant cases by paired t tests. A p value of 0.05 was accepted as statistically significant.

### Assessment of extracellular levels of biogenic amines

Four months old male mice were used for these experiments. The procedures used for the experiments described in this report received prior approval from the City of Basel Cantonal Animal Protection Committee based on adherence to federal and local regulations on animal maintenance and testing.

### Surgery and implantation of the microdialysisprobe

Forty-five minutes before anesthesia mice received subcutaneously 0.075 mg/kg of buprenorfine. Mice were then anesthetized with isoflurane and placed in a stereotaxic device equipped with dual manipulators arms and an anesthetic mask. Anesthesia was maintained with isoflurane 0.8-1.2% (v/v; support gas oxygen/air, 2:1). The head was shaved and the skin was cut along the midline to expose the skull. A small bore hole was made in the skull to allow the stereotaxical insertion of the microdialysis probe (vertical probe carrying a 2 mm polyacrilonitrile dialysis membrane; Brains On-line, Groningen, The Netherlands) in the striatum (coordinates: A 0.9 mm, L-1.8 mm, V -4.6 mm). The probe was cemented into place using binary dental cement. Once the cement was firm, the wound was closed with silk thread for suture (Silkam) the animal was removed from the stereotaxic instrument and returned to its cage. At the end of the surgery and 24 hrs later mice were treated with Meloxicam 1 mg/kg sc. The body weight of the animals was measured before the surgery and in the following days to monitor the recovery of the animal from surgery.

### Microdialysis experiments

All microdialysis experiments were carried out 3-4 days after surgery in awake, freely moving mice. The day of the experiment, the inlet of the implanted dialysis probe was connected to a micro-perfusion pump (CMA/Microdialysis, Sweden) and the outlet was connected to a fraction collector. The microdialysis probe was then perfused with Ringer solution (NaCl 147 mM, KCl 3 mM, CaCl₂ 1.2 mM, MgCl₂ 1.2 mM) at a constant flow rate of 1.5µl/min and dialyzates were collected in 15 min aliquots in plastic vial containing 37.5µl of acetic acid 0.02 M. Four samples of dialysates were collected before pharmacological treatment to determine the baseline levels of biogenic amines and their metabolites. Mice were then treated intraperitoneally with 2.5 mg/kg of amphetamine and dialysate samples collected for further 2.5 hrs. Dialysate samples were stored frozen at -80°C until analysis.

### Analysis of microdialysate

Frozen dialysate samples were shipped in dry ice to Brains On-Line for assay of monoamines and their metabolites. The concentrations of dopamine, DOPAC, serotonin, 5-HIAA and noradrenaline were measured by use an HPLC equipped with an electrochemical detector according to the procedure of van der Vegt et al. (2003).

### In vivo microdialysis assessment of biogenic amine neurotransmitter levels

Concentrations of norepinephrine, dopamine, and serotonin were determined within the same samples by HPLC separation and electrochemical detection. Samples were split into two aliquots; one used for simultaneous analysis of norepinephrine and dopamine, the other for analysis of serotonin.

### Norepinephrine and Dopamine

Aliquots (20 µL) were injected onto the HPLC column by a refrigerated microsampler system, consisting of a syringe pump (Gilson, model 402), a multi-column injector (Gilson, model 233 XL), and a temperature regulator (Gilson, model 832). Chromatographic separation was performed on a reverse-phase 150 x 2.1 mm (3 µm) C18 Thermo BDS Hypersil column (Keystone Scientific). The mobile phase (isocratic) consisted of a sodium acetate buffer (4.1 g/L Na acetate) with 2.5 % v/v methanol, 150 mg/L Titriplex (EDTA), 150 mg/L 1-octanesulfonic acid, and 150 mg/L tetramethylammonium chloride (pH = 4.1 adjusted with glacial acetic acid). Mobile phase was run through the system at a flow rate of 0.35 mL/min by an HPLC pump (Shimadzu, model LC-10AD vp).

Norepinephrine and dopamine were detected electrochemically using a potentiostate (Antec Leyden, model Intro) fitted with a glassy carbon electrode set at +500 mV vs. Ag/AgCl (Antec Leyden). Data were analyzed by Chromatography Data System (Shimadzu, class-vp) software. Concentrations of monoamines were quantitated by external standard method.

### Serotonin

Aliquots (20 µL) were injected onto the HPLC column as described for norepinephrine and dopamine. Chromatographic separation was performed on a reverse-phase 100 x 2 mm (3 µm) C18 ODS Hypersil column (Phenomenex). The mobile phase (isocratic) consisted of a sodium acetate buffer (4.1 g/L Na acetate) with 4.5 % v/v methanol, 500 mg/L Titriplex (EDTA), 50 mg/L 1-heptanesulfonic acid, and 30 µL/L tetraethylammonium (pH = 4.74 adjusted with glacial acetic acid). Mobile phase was run through the system at a flow rate of 0.4 mL/min by an HPLC pump (Shimadzu, model LC-10AD vp). Serotonin was detected electrochemically using the same method as described for norepinephrine and dopamine.

### Slice electrophysiology in the ventral tegmental area (VTA).

Horizontal slices (250µm thick, VT1000 vibratome, Leica) of the midbrain were prepared from TAAR1 knock-out and littermate wild-type mice 25-60 days of age. Slices were cooled in artificial cerebrospinal fluid (ACSF) containing in mM: 119 NaCl, 2.5 KCl, 1.3 MgCl₂, 2.5 CaCl₂, 1.0 NaH₂PO₄, 26.2 NaHCO₃ and 11 glucose. Slices were continuously bubbled with 95% O2 and 5% CO2 and transferred after 1 h to the recording chamber superfused (1.5 ml/min) with ACSF at 32-34 °C. The VTA was identified as the region medial to the medial terminal nucleus of the accessory optical tract. Visualized whole-cell current-clamp recording techniques were used to measure the spontaneous firing rate and holding currents of neurons. All cells used for the statistical analysis displayed a stable firing activity for more than 30 minutes. Dopaminergic neurons were identified by a large Ih current. The internal solution contained in mM: 140 potassium gluconate, 4 NaCl, 2 MgCl₂, 1.1 EGTA, 5 HEPES, 2 Na₂ATP, 5 sodium creatine phosphate and 0.6 Na₂GTP; the pH was adjusted to 7.3 with KOH. Data were obtained with an Axopatch 200B (Axon Instruments, Union City, CA, USA), filtered at 2kHz and digitized at 10kHz, acquired and analyzed with pClamp9 (Axon Instruments, Union City, CA, USA). Values are expressed as mean±sem. For statistical comparisons we used the Kolmogorov-Smirnov test. The level of significance was set at P=0.05.

### RESULTS

### Physical and behavioral properties of TAAR1^{LacZ/LacZ} mice

The general health, physical state and sensory functions of the TAAR1^{LacZ/LacZ} mouse line was examined according to a modified version of standard procedures used for behavioral phenotyping of genetically modified mice (Irwin, S. Comprehensive observational assessment: Ia. A systematic, quantitative procedure for assessing the behavioral and physiologic state of the mouse. Psychopharmacologia 13, 222-257 (1968); Hatcher, J.P. et al. Development of SHIRPA to characterise the phenotype of genetargeted mice. Behav. Brain Res. 125, 43-47 (2001)). The comparison of TAAR1^{+/LacZ} and TAAR1^{LacZ/LacZ} mice to their wild-type siblings did not reveal any significant differences regarding their general state of health, their viability, fertility, lifespan, nest building behaviour (Fig. 12a), body weight (Fig. 12b) as well as their body temperature (Fig. 12c). Regarding general motor functions and behavior no significant differences between genotypes were observed analyzing dexterity and motor coordination (Fig. 12d-f) as well as spontaneous locomotor activity (Fig. 12g).

### TAAR1^{LacZ/LacZ} mice display elevated sensitivity to psychostimulants

Recent observations indicate that at least part of the pharmacological effects of trace amines are due to modulation of catecholamine neurotransmission (Berry, M.D. Mammalian central nervous system trace amines. Pharmacologic amphetamines, physiologic neuromodulators. J. Neurochem. 90, 275-271 (2004); Geracitano, R., Federici, M., Prisco, S., Bernardi, G. & Mercuri N.B. Inhibitory effects of trace amines on rat midbrain dopaminergic neurons. Neuropharmacol. 46, 807-814 (2004)). In addition, TAAR1 has been found to be localized in brain areas with pronounced dopaminergic and serotonergic neurotransmission (Borowsky, B. et al. Trace amines: identification of a family of mammalian G protein-coupled receptors. Proc. Natl. Acad. Sci. USA 98, 8966-8971 (2001)). Amphetamines are known to act as indirect catecholamine agonists that achieve their pharmacological effects by inducing the release of cytosolic dopamine and norephinephrine (King, G.R. & Ellinwood, E.H. Amphetamines and other stimulants. In Lowinson, J.H., Ruiz, P., Millman, R.B. & Langrod, J.G., editors. Substance abuse: a comprehensive textbook, Williams & Wilkins., Baltimore, 1992). Increased extracellular levels of these neurotransmitters, in turn, produce hyperlocomotor activity. The effect of d-amphetamine (2.5 mg/kg i.p.) on locomotor function was therefore compared between TAAR1^{LacZ/LacZ} and TAAR1^{+/+} mice. Whereas the locomotor activity decreased in wild type mice after d-amphetamine injection regarding total distance moved TAAR1^{LacZ/LacZ} mice were first more active and then moved significantly more than wild type littermates (Fig. 12g). Similar results were obtained looking at horizontal activity and stereotypie (results not shown). Basal locomotor activity before amphetamine application was comparable between both genotypes (Fig. 12g).

The behavioural changes were further investigated in microdialysis studies. The effect of d-amphetamine on the extracellular levels of catecholamines in the striatum revealed 2.3 fold increased levels of dopamine and norepinephrine in TAAAR1^{LacZ/LacZ} compared to wild type mice (Fig. 13a and 13c). No significant differences in basal levels of dopamine (2.23 +/- 0.65 □M and 2.27 +/- 0.68µM in TAAR1^{+/+} and TAAR1^{LacZ/LacZ}, respectively) and norepinephrine (0.30 +/- 0.12µM and 0.38 +/- 0.18µM in TAAR1^{+/+} and TAAR1^{LacZ/LacZ}, respectively) were seen. In TAAR1^{LacZ/LacZ} mice dopamine and norepinephrine levels increased by 11 and 4.9 fold, respectively. Whereas no significant changes in the basal level of the dopamine metabolite DOPAC have been seen in TAAR1^{LacZ/LacZ} mice (basal level: 148 +/- 36µM), DOPAC levels were significantly decreased versus wild-type control 45 min after d-amphetamine administration and returned to basal levels after 135 minutes in TAAR1^{LacZ/LacZ} mice (Fig. 13b; basal level: 132 +/- 44µM). Serotonin levels remained unchanged after d-amphetamine application in wild type animals (basal level: 0.35µM), but increased by 2.5 fold in TAAR1^{LacZ/LacZ} mice. No significant changes were seen in levels of the 5-HT metabolite 5-HIAA in both genotypes (basal levels: 124 +/- 19µM in TAAR1^{+/+} and 119 +/- 18µM in TAAR1^{LacZ/LacZ}).

### TAAR1 activity decreases the spontaneous firing rate of dopaminergic neurons in the VTA

The spontaneous firing rate of dopaminergic neurons in the VTA was determined under current clamp conditions. The mean spike frequency in TAAR1^{+/+} (n = 22) and in TAAR1^{LacZ/LacZ} (n = 25) was 2.3±0.8 Hz and 17.2±1.2 Hz (p<0.0001, Fig. 14a), respectively, thus revealing a significantly increased firing rate in TAAR1^{LacZ/LacZ} neurons. The data suggest that in wild type mice TAAR1 is tonically activated by ambient concentrations of an endogenous ligand. We further observed that the resting membrane potential in the TAAR1^{LacZ/LacZ} mice (-33.53±0.55 mV, n = 26) was depolarized compared to wild-type mice (-47.82±0.66 mV, n = 22). The depolarized resting membrane potential may to some extent underlie the increased firing rate but alternatively could also be a consequence of the increased firing rate. We next tested whether application of p-tyramine decreases the spontaneous firing rate of dopaminergic neurons in the VTA of wild type mice. Bath application of p-tyramine (10µM) caused a significant decrease in the spike frequency in TAAR^{+/+} (control: F = 2.1±0.3 Hz, p-tyramine: F = 0.63±0.04 Hz, n = 19, p<0.0001) but not in the TAAR1^{LacZ/LacZ} mice (control: F = 16.73±1.15 Hz, p-tyramine: F = 16.57±1.35 Hz, n = 15, p>0.05; Fig. 14b). This directly shows that TAAR1 activity can inhibit the spontaneous firing of dopaminergic neurons in the VTA.

## Claims

1. A vector construct comprising genomic sequences homologous to upstream and downstream regions flanking the single coding exon of the TAAR1 gene suitable for homologous recombination and one or more selection marker genes.

2. A vector construct according to claim 1 wherein the vector construct comprises additionally a reporter gene, and wherein the reporter gene is located between the homologous TAAR1 flanking sequences.

3. The vector construct according to claim 2 wherein the reporter gene encodes LacZ.

4. The vector construct according to any one of claims 2 to 3 wherein the reporter gene is operably linked to a NLS sequence.

5. The vector construct according to any one of claims 1 to 4, wherein the selection marker gene is a neomycin resistance gene.

6. The vector construct according to any one of claims 1 to 5, wherein the selection marker gene is a diphtheria toxin gene.

7. The vector construct according to any one of claims 1 to 6, wherein said selection marker genes are a neomycin resistance gene and a diphtheria toxin gene.

8. The vector construct according to any one of claims 1 to 7 wherein the homologous sequences comprise at least a part of the TAAR1 promoter.

9. A vector construct TAAR-KO incorporated in the plasmid pSKDT-Tar1-NLS-PGK-Neo deposited under the accession number DSM 17504.

10. A method of producing a non-human knock-out animal, whose one or both alleles of *TAAR1* gene are mutated and/or truncated in a way that less or no active TAAR1 protein is expressed comprising
(a) introducing a vector construct according to any one of claims 1 to 9 into an embryonic stem cell by means of homologous recombination,
(b) generating a heterozygous and/or homozygous knock-out animal from the said embryonic stem cell, and thereby
(c) producing a non-human knock-out animal, whose one or both alleles of a *TAAR1* gene are mutated and/or truncated in a way that less or no active TAAR1 protein is expressed.

11. The method according to claim 10, wherein the embryonic stem cell of a) is derived from the mouse strain C57BL/6.

12. A non-human knock-out animal produced by the method according to any one of claims 10 to 11.

13. A non-human knock-out animal whose one or both alleles of a *TAAR1* gene are mutated and/or truncated in a way that less or no active TAAR1 protein is expressed.

14. The non-human knock-out animal according to claim 13 wherein one or both alleles of a *TAAR1* gene are replaced by a reporter gene.

15. The non-human knock-out animal according to claim 14, wherein the reporter gene encodes LacZ.

16. The non-human knock-out animal according to any one of claims 12 to 15, wherein the animal is a rodent.

17. The non-human knock-out animal according to claim 16, wherein the rodent is a mouse.

18. The non-human knock-out animal according to claim 17, wherein the mouse is a co-isogenic C57BL/6 mouse.

19. Descendant of the non-human knock-out animal according to any one of claims 12 to 18, obtained by breeding with animals of the same or another genotype.

20. A primary cell culture or a secondary cell line derived from a non-human knock-out animal or its descendants according to any one of claims 12 to 19.

21. A tissue or organ explant or culture thereof, derived from a non-human knock-out animal or its descendants according to claims 12 to 19.

22. A tissue or cell extract derived from a non-human knock-out animal or its descendants according to claims 12 to 19.

23. Use of a non-human knock-out animal according to claims 12 to 19, or primary cell culture or secondary cell line according to claim 20, or a tissue or organ explant or culture thereof according to claim 21, or a tissue or cell extract according claim 22 as a model for identifying and testing the therapeutic effect of a compound in disorders comprising depression, anxiety disorders, bipolar disorder, attention deficit hyperactivity disorder, stress-related disorders, psychotic disorders such as schizophrenia, neurological diseases such as Parkinson's Disease, neurodegenerative disorders such as Alzheimer's Disease, epilepsy, migraine, hypertension, substance abuse and metabolic disorders such as eating disorders, diabetes, diabetic complications, obesity, dyslipidemia, disorders of energy consumption and assimilation, disorders and malfunction of body temperature homeostasis, disorders of sleep and circadian rhythm, and cardiovascular disorders.

24. Use of a non-human knock-out animal according to claims 12 to 19, or a primary cell culture or secondary cell line according to claim 20, or a tissue or organ explant or culture thereof according to claim 21, or a tissue or cell extract according claim 22 as a tool for assessing TAAR1 function.

25. Use of a non-human knock-out animal according to claims 12 to 19, or primary cell culture or secondary cell line according to claim 20, or a tissue or organ explant or culture thereof according to claim 21, or a tissue or cell extract according claim 22 as a tool for identifying unknown ligands of TAAR1 and for the characterization of ligands to TAARs other than TAAR1.

26. A method of testing TAAR1 inhibitor compounds for effects other than TAAR1-specific effects which method comprises administering a TAAR1 agonist, a TAAR1 partial agonist, a TAAR1 modulator or a TAAR1 inhibitor compound to a non-human knock-out animal according to claims 12 to 19, or primary cell culture or secondary cell line according to claim 20, or a tissue or organ explant or culture thereof according to claim 21, or a tissue or cell extract according claim 22, and determining the effect of the compound by behavior and physiological studies addressing neurological, sensory, and cognitive functions as well as physiological parameters and comparing these to the effect(s) of the same compound on wild type control animals.

27. Use of a non-human knock-out animal according to claims 12 to 19, or a primary cell culture or secondary cell line according to claim 20, or a tissue or organ explant or culture thereof according to claim 21, or a tissue or cell extract according claim 22 as a tool for testing a TAAR1 agonist, a TAAR1 partial agonist, a TAAR1 modulator or a TAAR1 inhibitor compounds for effects other than TAAR1-specific effects.

28. Use of a non-human knock-out animal according to claims 12 to 19, or a primary cell culture or secondary cell line according to claim 20, or a tissue or organ explant or culture thereof according to claim 21, or a tissue or cell extract according claim 22 as a tool for determining the specificity of compounds acting on TAAR1.

29. Use of a non-human knock-out animal according to claims 12 to 19, or a primary cell culture or secondary cell line according to claim 20, or a tissue or organ explant or culture thereof according to claim 21, or a tissue or cell extract according claim 22 for studying the intracellular trafficking of TAARs or of other cellular components linked to TAARs.

30. Use of the non-human knock-out animal, according to any one of the claims 14 to 19 for determining the TAAR1 expression profile.

31. A test system for testing TAAR1 agonists, TAAR1 partial agonists, TAAR1 positive and negative modulators or TAAR1 inhibitor compounds for effects other than TAAR1-specific effects comprising non-human knock-out animal according to claims 12 to 19, or a primary cell culture or secondary cell line according to claim 20, or a tissue or organ explant or culture thereof according to claim 21, or a tissue or cell extract according claim 22, and a means for determining whether TAAR1 agonists, TAAR1 partial agonists, TAAR1 positive and negative modulators or TAAR1 inhibitor compounds exhibit effects other than TAAR1-specific effects.

32. The vector constructs, methods, knock-out animals, test systems and uses substantially as described herein before especially with reference to the foregoing examples.


SEQUENCE LISTING

<110> F. Hoffmann-La Roche AG

<120> TAAR1 Knock-out animal

<130> 23185

<160> 29

<170> PatentIn version 3.3

<210> 1
<211> 546
<212> DNA
<213> Mus musculus

<400> 1
gagggaaagc ccagcctgtg tctagttctc tgcagtgatg catctttgcc acgctatcac 60

aaacatttcc cacagaaaca gcgactggtc aagagaagtc caagcttccc tgtacagctt 120

aatgtcactc ataatcctgg ccactctggt tggcaactta atagtaataa tttccatatc 180

ccatttcaag caacttcata cacccaccaa ctggctcctt cactccatgg ccattgtcga 240

ctttctgctg ggctgtctga taatgccctg cagcatggtg agaactgttg agcgctgttg 300

gtattttggg gaaatcctct gtaaagttca caccagcacc gatatcatgc tgagctccgc 360

ctccattttc cacttagctt tcatttccat tgaccgctac tgtgctgtgt gtgacccttt 420

gagatacaaa gccaagatca atatctccac tattcttgtg atgatcctcg ttagttggag 480

ccttcctgct gtttatgcat ttgggatgat cttcctggaa ctgaacttaa aaggagtgga 540

agagct 546

<210> 2
<211> 742
<212> DNA
<213> Mus musculus

<400> 2
gacattttat ttacaggcac agagtctcct ggacaggctg gtgaaggtga actctgattc 60

aaagaccgtg tgaggggtat ccaccagaga agaccgctgg gaccagggct taagtcttta 120

ttagcaggtc ggtgtctaca ctgggtgttt gggatcccag tgtagtcccg agcctttctt 180

tgagccttta agcacaaaaa aaaaccaaaa aactagtttc agggttgaca tacttcagtt 240

accaagaaca gttagccaga agtggaacta taaaagccaa atagtaaggt taatacattt 300

ggaaactttc ccaggccttt gatggattag gtctgtgttt tagttttggc agccagtgct 360

atgtgctgag tttggcagcc tgagtgatgt gtctatcatg gagtccatca tgctaaggtg 420

tcagggtcta ctaaagtctg tggggctgtt acaaggccat attcatgtaa aacctacaca 480

aacgtgccat tctataaaag acggttacat aacaggtata tcatgattta ctttcgggtt 540

ctctagatat ctggtgtcag tcattttgtg ctgactcatg gtggtcctga aaaagtataa 600

cgtaaaagca aaattctagg cctttaggcc caggcttgag aatgtgacct ctgtgactca 660

atgctccaag gtatgctaat cataaaacag atagcgacat tcctccaccc accctcttcc 720

tgggttcaag gagtgttcat tt 742

<210> 3
<211> 873
<212> DNA
<213> Mus musculus

<400> 3
tgttcttctc taagagcctg tcactcaccg gcattcggca accttcatcg ttgttggttt 60

gtaaacaagt gtggggatgc tctttgacat tttcatttct ataatgttta gtacgttcac 120

tgtccatcag atcagtatga tatttagtca cgatttcatt attcgctttt gtactttcct 180

ggagaaatta aaacaccaca taccatttct ttagaccatt cacatttatc ttcttagtta 240

gtgtgcttta tctgtttttg gctttagatt ttcatttctc tgtctccaca gtcctttctt 300

atttgacttg gcttctgtct gctgtcattc cacattgcta atggtgtttt aatgtttctt 360

ggcaacacat ctcaggagca cgggatttgg agcatctcta agattctgct atcggggtct 420

cttcagtaga ttctctttca ctgacaaagc ttactgtcag tgcttgagag ctcactccac 480

acttggttgt tacacatgca gctctcccgt aattgcattt gtattttaag gcagaacttg 540

ttgcaattat tgaaacacaa ttctctttga tttgcataga attaattatt tggttttgtt 600

tagaacccag ttctcaacat cttaattttc ctgtcttctt caagcaataa aggacacata 660

ggtgttgtat cttgtgattc cttgattctg agagacatct ttcacactta cacaaagaca 720

tttctttgtt ctttgttaaa tattcttaca tttaacattc tcttgtttta aacaagttcc 780

ttgtagctct ctatgttgtt tcttattaaa tgaaaggctc agcattcctg agacatttaa 840

ggctatgtga ctctgggaaa acacattaat taa 873

<210> 4
<211> 547
<212> DNA
<213> Artificial

<220>
<223> Description of Artificial Sequence: Sequence comprises murine
      sequence and NLS and part of DNA encoding LacZ (E.coli).

<400> 4
gggaaagccc agcctgtgtc tagttctctg cagtgatgca tccaaagaag aagagaaagg 60

tttcggaggg ggggagcttg atgatctgtg acatggcgga tcccgtcgtt ttacaacgtc 120

gtgactggga aaaccctggc gttacccaac ttaatcgcct tgcagcacat ccccctttcg 180

ccagctggcg taatagcgaa gaggcccgca ccgatcgccc ttcccaacag ttgcgcagcc 240

tgaatggcga atggcgcttt gcctggtttc cggcaccaga agcggtgccg gaaagctggc 300

tggagtgcga tcttcctgag gccgatactg tcgtcgtccc ctcaaactgg cagatgcacg 360

gttacgatgc gcccatctac accaacgtga cctatcccat tacggtcaat ccgccgtttg 420

ttcccacgga gaatccgacg ggttgttact cgctcacatt taatgttgat gaaagctggc 480

tacaggaagg ccagacgcga attatttttg atggcgttaa ctcggcgttt catctgtggt 540

gcaacgg 547

<210> 5
<211> 279
<212> DNA
<213> Artificial

<220>
<223> Description of Artificial Sequence: Sequence comprises murine
      sequence and NLS and part of DNA encoding LacZ (E.coli)..

<400> 5
gagggaaagc ccagcctgtg tctagttctc tgcagtgatg catccaaaga agaagagaaa      60

ggtttcggag ggggggagct tgatgatctg tgacatggcg gatcccgtcg ttttacaacg     120

tcgtgactgg gaaaaccctg gcgttaccca acttaatcgc cttgcagcac atcccccttt     180

cgccagctgg cgtaatagcg aagaggcccg caccgatcgc ccttcccaac agttgcgcag     240

cctgaatggc gaatggcgct ttgcctggtt tccggcacc                            279


<210> 6
<211> 1243
<212> DNA
<213> Artificial

<220>
<223> Description of Artificial Sequence: Sequence contains murine
      sequence and part of PGK-NEO-Resistance cassette.

<400> 6
tcatctccgg gcctttcgac ctgcagccaa tatgggatcg gccattgaac aagatggatt      60

gcacgcaggt tctccggccg cttgggtgga gaggctattc ggctatgact gggcacaaca     120

gacaatcggc tgctctgatg ccgccgtgtt ccggctgtca gcgcaggggc gcccggttct     180

ttttgtcaag accgacctgt ccggtgccct gaatgaactg caggacgagg cagcgcggct     240

atcgtggctg gccacgacgg gcgttccttg cgcagctgtg ctcgacgttg tcactgaagc     300

gggaagggac tggctgctat tgggcgaagt gccggggcag gatctcctgt catctcacct     360

tgctcctgcc gagaaagtat ccatcatggc tgatgcaatg cggcggctgc atacgcttga     420

tccggctacc tgcccattcg accaccaagc gaaacatcgc atcgagcgag cacgtactcg     480

gatggaagcc ggtcttgtcg atcaggatga tctggacgaa gagcatcagg ggctcgcgcc     540

agccgaactg ttcgccaggc tcaaggcgcg catgcccgac ggcgatgatc tcgtcgtgac     600

ccatggcgat gcctgcttgc cgaatatcat ggtggaaaat ggccgctttt ctggattcat     660

cgactgtggc cggctgggtg tggcggaccg ctatcaggac atagcgttgg ctacccgtga     720

tattgctgaa gagcttggcg gcgaatgggc tgaccgcttc ctcgtgcttt acggtatcgc     780

cgctcccgat tcgcagcgca tcgccttcta tcgccttctt gacgagttct tctgagggga     840

tcaattctct agagctcgct gatcagcctc gactgtgcct tctagttgcc agccatctgt     900

tgtttgcccc tcccccgtgc cttccttgac cctggaaggt gccactccca ctgtcctttc     960

ctaataaaat gaggaaattg catcgcattg tctgagtagg tgtcattcta ttctgggggg    1020

tggggtgggg caggacagca agggggagga ttgggaagac aatagcaggc atgctgggga    1080

tgcggtgggc tctatggctt ctgaggcgga aagaaccagc tggggctcga tcctctagag    1140

tcgatcgacc tcgacccccc ctatgcattt ttggagaatc atctatactt tacagtgagt    1200

ttctgtggct ttatttattt caagatcttc cttacagatt att 243

<210> 7
<211> 738
<212> DNA
<213> Artificial

<220>
<223> Description of Artificial Sequence: Sequence contains murine
      sequence and part of PGK-NEO-Resistance cassette.

<400> 7
cgatcgacct cgaccccccc tatgcatttt tggagaatca tctatacttt acagtgagtt 60

tctgtggctt tatttatttc aagatcttcc ttacagatta tttccagatg ttgtataaat 120

cttttaaaaa ctgtggtact gattcacatg agaaaaaagg ctctagaaat gaagagccca 180

agatccagaa ataactgtct gggtttatga gcagttgtgt agctgtgtga tcagaggttg 240

attatgtaca gtctaagggg agtagctgag tgaaaaggaa aaagactagt ttttaatgta 300

accattattg ccactattat atatgttata taaacaaggt tatgtataaa caataaagtt 360

tatattatat tttcacttgt tttgtttctt gttttataat atataggcat attaaacttt 420

acattgtttt aacttttttt aaataattgc tattattttt ttgagacagg gtcttagtat 480

atagcttgac tagcccagaa gtctctgtga agactaggat ggtctcagac tcagagagat 540

ttgcctgccc ctgctgccca agtgctgaga tttaagtatt tagtcattaa ggtttgtata 600

tacattatgt ctagccattg aggtttttat attcaccaca tccagatgtt aaggtttcta 660

ggcacacatc catttcttac tctattagtc agaatttcta gaacaatact gatattaagg 720

atggatctta ttacttgg 738

<210> 8
<211> 796
<212> DNA
<213> Mus musculus

<400> 8
cctacctctg gcctctggcc tttcccttta actccacaga taagtgcata agctccttta 60

aaaatttcag aagatttttt ttccagaatc tttataaatg taagcaggca gtgatccttc 120

acattatcat taggagggac tagccaaagg gtgtagagtt ccagtttgca caagggctaa 180

ggtcatggtc agacagctcc ctctatgtcc atttccaatg tgagttacga aacttcttac 240

caagttcatg agttccagac agccttccaa gaaaagtttt tagttacatc aatggaaaag 300

atagaatctg gttgttgcaa gaaaaacagt tacaacaaca tcaatactta tttctaagtc 360

aatttgtggc aaaaataatt tcatttcgca ggttctactg aaaatattca tataaatgtt 420

catttcatat caagtagtaa tgtgttggtc aggacttttt tgtggaaatg gattattaac 480

aacggtgctt tcttaagagg ttagtgagct aggaggttcc atcatgattt tagactatat 540

ctggaagtca acaatccagt cttggcaaac tcacacatat ttgtcagaaa gtaggacaaa 600

gtgggtaatc tccaatccac tgagaatttt ggtaagtcac agtttctgct tagctgaacc 660

agagtcaaga agagcttgaa tattaagggt agaaagatgc atgaagaatg gtggatttgg 720

tttttctaaa acagtttgct ttgaatattt attttagtta ttttttgggg ggaggaaagt 780

ttatttagct tacact 796

<210> 9
<211> 779
<212> DNA
<213> Mus musculus

<400> 9
ggcattcccc tgtactgggg catataaagt ttgcaatacc aaaggtcctc tcctcccagt 60

gatggccgat taggccatct tctgctacat atgcagctag agatatgaag tcattagata 120

tgcactcact gataagtgga tattagccca gaaacataga acacccaaga tacaatttgc 180

aaaacacaag aaaatcaaga agaaggaaga ccaatggatg gatacttcat tcctccttag 240

aatagggaac aaaataccca tgaaaggagt tacagagaca aagtttggag ctaagacaaa 300

aggatggact atccagagac tagccctccc agggatccat cccataatca gccaccaaac 360

ctagacacta ttgcatatgc cagaaagatt ttgctgaagg gaccctgtta tagctgtctc 420

gtatgaggct atgccagtgc ctggcaaaca cagaagtgga tgctcacagt catctataag 480

atggaactca gggcccccaa tggaggagca agagaaagca cccaagaaga tgaaggggtc 540

tgcaacccta taggtggaac aacaatatga actaaccagt acccccagag ctcatatctc 600

tagctgcata tattagttat tttaaagttg tgtgtgcatg catgtgtgtg cacgtgggaa 660

cagaagtgtg ggtgccagta gaggaatttc aatcccttgc agctagaatt acagggagac 720

ttgaattgcc cacaagattg ctgggaactg actctgggtc ctcagtaaga gctgcattt 779

<210> 10
<211> 28
<212> DNA
<213> Artificial

<220>
<223> Description of Artificial Sequence: Oligonucleotide derived from
TAAR1 (mus musculus) genomic sequence, additional restriction
site inserted.

<400> 10
cgggtacctg tcactcaccg gcattcgg 28

<210> 11
<211> 22
<212> DNA
<213> Mus musculus

<400> 11
ccttgcttgt cctttagcta tg 22

<210> 12
<211> 23
<212> DNA
<213> Mus musculus

<400> 12
cccatgtgac caatttgttc acc 23

<210> 13
<211> 35
<212> DNA
<213> Artificial

<220>
<223> Description of Artificial Sequence: derived from TAAR1 genomic
      sequence (mus musculus), additional restriction site inserted.

<400> 13
ctgctgcccg ggattcaagc tcttcttgac tctgg                                35


<210> 14
<211> 37
<212> DNA
<213> Artificial

<220>
<223> Description of Artificial Sequence: Oligonucleotide for
      introducing NLS and cloning site StuI in vector construct

<400> 14
tccaaagaag aagagaaagg tttcggaggc ctatgca                              37


<210> 15
<211> 37
<212> DNA
<213> Artificial

<220>
<223> Description of Artificial Sequence: Oligonucleotide for
introducing NLS and cloning site StuI in vector construct.

<400> 15
ttggcctccg aaacctttct cttcttcttt ggatgca                              37


<210> 16
<211> 20
<212> DNA
<213> Escherichia coli

<400> 16
gcgcatcgcc ttctatcgcc                                                 20


<210> 17
<211> 22
<212> DNA
<213> Mus musculus

<400> 17
gagcttcaca catgaacaca cc                                              22


<210> 18
<211> 21
<212> DNA
<213> Mus musculus

<400> 18
gtgggctaag atctaggaac g                                               21


<210> 19
<211> 20
<212> DNA

<213>  Escherichia coli

<400>  19
ggcgatagaa ggcgatgcgc                                                    20


<210>  20
<211>  22
<212>  DNA
<213>  Mus musculus

<400>  20
gaaggtggaa ttctaacctg ac                                                 22


<210>  21
<211>  22
<212>  DNA
<213>  Mus musculus

<400>  21
ccttgcttgt cctttagcta tg                                                 22


<210>  22
<211>  20
<212>  DNA
<213>  Escherichia coli

<400>  22
cttgggtgga gaggctattc                                                    20


<210>  23
<211>  20
<212>  DNA
<213>  Escherichia coli

<400>  23
aggtgagatg acaggagatc                                                    20


<210>  24
<211>  20
<212>  DNA
<213>  Mus musculus

<400>  24
accacagtcc atgccatcac                                                    20


<210>  25
<211>  20
<212>  DNA
<213>  Mus musculus

<400>  25
tccaccaccc tgttgctgta                                                    20


<210>  26
<211>  21
<212>  DNA
<213>  Mus musculus

<400>  26
atgcatcttt gccacgctat c                                                  21


<210>  27

<211>  20
<212>  DNA
<213>  Mus musculus

<400>  27
caaggctctt ctgaaccagg                                                    20


<210>  28
<211>  18
<212>  DNA
<213>  Escherichia coli

<400>  28
ggtggcgctg gatggtaa                                                      18


<210>  29
<211>  18
<212>  DNA
<213>  Escherichia coli

<400>  29
cgccatttga ccactacc                                                      18